(19) Europäisches Patentamt
European Patent Office
Office européen des brevets

(11) EP 4 260 912 A1

(12) # EUROPEAN PATENT APPLICATION
published in accordance with Art. 153(4) EPC

(43) Date of publication:
**18.10.2023 Bulletin 2023/42**

(21) Application number: **21903133.3**

(22) Date of filing: **17.11.2021**

(51) International Patent Classification (IPC):
***A61Q 11/00*** *(2006.01)* ***A61K 8/02*** *(2006.01)*
***A61K 8/19*** *(2006.01)* ***A61K 8/25*** *(2006.01)*
***A61K 8/34*** *(2006.01)* ***A61K 8/44*** *(2006.01)*
***A61K 8/60*** *(2006.01)*

(52) Cooperative Patent Classification (CPC):
**A61K 8/02; A61K 8/19; A61K 8/25; A61K 8/34; A61K 8/44; A61K 8/60; A61Q 11/00**

(86) International application number:
**PCT/JP2021/042276**

(87) International publication number:
**WO 2022/124029 (16.06.2022 Gazette 2022/24)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **11.12.2020 JP 2020205830**

(71) Applicant: **Kabushiki Kaisha Shofu**
**Kyoto-shi**
**Kyoto 605-0983 (JP)**

(72) Inventors:
- **NAKATSUKA, Toshiyuki**
  **Kyoto-shi**
  **Kyoto 605-0983 (JP)**
- **KASABA, Hideto**
  **Kyoto-shi**
  **Kyoto 605-0983 (JP)**

(74) Representative: **Eisenführ Speiser**
**Patentanwälte Rechtsanwälte PartGmbB**
**Postfach 31 02 60**
**80102 München (DE)**

(54) **POWDER MIXTURE FOR SPRAYING INTO TOOTH SURFACE OR INTO GINGIVAL SULCUS/PERIODONTAL POCKET**

(57) A spray powder mixture to be sprayed to a tooth surface above or below a gingival margin or into a gingival sulcus and a periodontal pocket by a powder spraying apparatus, the spray powder mixture comprising:
(a) at least one component selected from a group consisting of a saccharide, a sugar alcohol, an amino acid, a phosphate compound, a carbonate compound, and a calcium compound; and
(b) a hydrophobized particulate silica.

FIG. 2

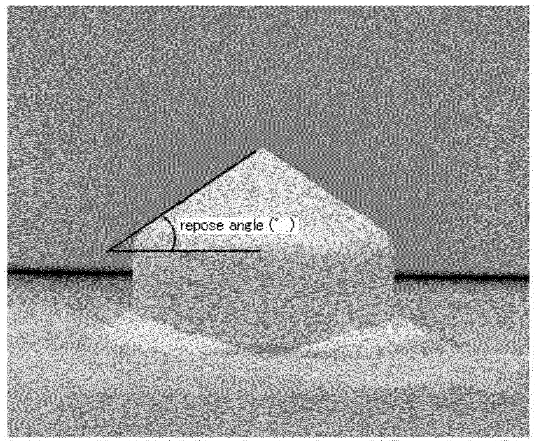


EP 4 260 912 A1

## Description

### TECHNICAL FIELD

The present invention relates to a spray powder mixture, particularly a spray powder mixture to be sprayed to a tooth surface above or below a gingival margin or into a gingival sulcus and a periodontal pocket after being mixed with air in a powder/air mixing chamber of a powder spraying apparatus.

### BACKGROUND ART

The periodontal disease refers to all diseases occurring in periodontal tissues consisting of gingiva, dental cementum, periodontal membrane and alveolar bone, and is an inflammatory disease mainly caused by oral bacteria in plaque, and is roughly classified into gingivitis and periodontitis. The periodontal disease is referred to as "gingivitis" when inflammation is confined to the gingiva, and the periodontal disease is referred to as "periodontitis" when inflammation sites spread beyond the gingiva to the periodontal membrane and alveolar bone, and the affected sites are damaged and destroyed. The gingivitis can be healed by mechanically removing causative plaque because inflammation does not spread to periodontal membrane or alveolar bone even if inflammation is observed in periodontal tissues. However, if the symptom progresses from gingivitis to periodontitis, tooth movement or damage or destruction of periodontal membrane and alveolar bone occurs, and if the symptom becomes more severe, it is difficult to recover the underlying health by mechanically removing plaque.

In recent years, the periodontal disease has been positioned as a lifestyle-related disease, affected approximately 80% of adults, and become the largest cause of tooth loss in adults. From recent studies, the periodontal disease has been indicated to be associated with serious systemic diseases (diabetes, arteriosclerosis, heart disease, premature birth, delivery of a low weight infant, cerebrovascular disease and the like), and revealed to become a risk factor. Further, the characteristics of periodontal disease are inflammation of soft tissues surrounding teeth, exposure of tooth root surfaces, formation of periodontal pockets, and progressive collapse of fibrous organs and alveolar bone supporting the teeth. If the periodontal disease is left untreated, the teeth will be eventually lost. While dental caries and periodontal disease have been said to be two major dental diseases, there has been a gradual decrease in the number of cases of dental caries, whereas the number of cases of periodontal disease has not tended to decrease because it is a refractory disease, and countermeasures against the periodontal disease are regarded as an important and urgent problem in preventive medicine. For this reason, it is important that periodontal disease is detected as early as possible, and immediately treated to prevent an increase in severity.

For preventing periodontal disease, it is necessary that plaque and calculus firmly stuck not only on a tooth surface above the gingival margin but also on narrow sites in gingival sulcus and periodontal pockets below the gingival margin be removed as much as possible. In the periodontal treatment, mechanical cleaning has been heretofore performed mainly using a curette, a sound wave, or an ultrasonic scaler. However, the use of such a device requires an advanced skill of a user, and it cannot be denied that the devices gives a sense of discomfort to a patient. In addition, repetition of such mechanical cleaning has abraded the tooth root, resulting in a risk of causing the onset of hypersensitivity or weakening of the tooth root.

It has been reported that a spray powder mixture is sprayed from a powder spraying apparatus together with water to clean a tooth surface for preventing and treating the above-mentioned periodontal disease (for example, US Patent No. 4174571, US Patent No. 4595365, JP-A-4418113, US Patent No. 6126444, US Patent No. 5810587, and JP-A-5846720).

### PATENT DOCUMENT (RELATED ART PATENT DOCUMENT)

Patent Document 1: US Patent No. 4174571
Patent Document 2: US Patent No. 4595365
Patent Document 3: JP-B2-4418113
Patent Document 4: US Patent No. 6126444
Patent Document 5: US Patent No. 5810587
Patent Document 6: JP-B2-5846720

## SUMMARY OF THE INVENTION

## PROBLEMS TO BE SOLVED BY THE INVENTION

However, the above-described spray powder mixture of the conventional arts do not enable sufficient prevention of damage to a tooth surface while having excellent fluidity allowing the spray powder mixture to easily discharged from a powder spraying apparatus.

For solving the various problems in the conventional arts, an object of the present invention is to provide a spray powder mixture for a powder spraying apparatus, which has further excellent fluidity in discharge from a powder spraying apparatus, and a low-abrasive property of hardly damaging a tooth surface.

## MEANS FOR SOLVING THE PROBLEMS

That is, the present invention provides a spray powder mixture to be sprayed to a tooth surface above or below a gingival margin or into a gingival sulcus and a periodontal pocket by a powder spraying apparatus, the spray powder mixture comprising:

(a) at least one component selected from a group consisting of a saccharide, a sugar alcohol, an amino acid, a phosphate compound, a carbonate compound, and a calcium compound; and
(b) a hydrophobized particulate silica.

## EFFECTS OF THE INVENTION

According to the present invention, it is possible to provide a powder mixture composition for a powder spraying apparatus, which has further excellent fluidity in discharge from a powder spraying apparatus, and a low-abrasive property of hardly damaging a tooth surface.

## BRIEF DESCRIPTION OF THE DRAWINGS

Fig. 1 shows a repose angle measuring device.
Fig. 2 shows a repose angle.

## MODES FOR CARRYING OUT THE INVENTION

Hereinafter, a spray powder mixture which is an embodiment for implementing the present invention will be described.

Numerical value ranges mentioned herein are intended to also include lower and upper limit values themselves unless otherwise specified by terms "less than" "smaller than or larger than" and the like. That is, a numerical value range of, for example, 1 to 100 is interpreted as including the lower limit value "1" and the upper limit value "100".

[Action Mechanism]

The embodiment of the present invention (hereinafter, referred to as the present embodiment) is a powder mixture to be sprayed to a tooth surface above or below the gingival margin or into a gingival sulcus and a periodontal pocket by a powder spraying apparatus, the spray powder mixture comprising:

(a) at least one component selected from a group consisting of a saccharide, a sugar alcohol, an amino acid, a phosphate compound, a carbonate compound, and a calcium compound; and
(b) a hydrophobized particulate silica.

The spray powder mixture according to the present embodiment is further excellent in both fluidity and low-abrasive property. The reason for this is, without being bound by a specific theory, is presumed as follows. The spray powder mixture according to the present embodiment comprises (a) at least one selected from the group consisting of a saccharide, a sugar alcohol, an amino acid, a phosphate compound, a carbonate compound, and a calcium compound, and (b) hydrophobized particulate silica. Here, the component (a) has relatively high hydrophilicity, and thus the components (a) tend to be aggregated, but the component (b) is present between the components (a), so that the components

(a) are inhibited from being aggregated to considerably increase the particle diameter (size). Thus, the spray powder mixture according to the present embodiment is further excellent in fluidity. Further, since an increase in the particle diameter of the component (a) is suppressed, impact on a tooth surface can be reduced. Thus, the spray powder mixture according to the present embodiment is further excellent in low-abrasive property. Therefore, the spray powder mixture according to the present embodiment may have both further excellent in both fluidity and low-abrasive property.

Here, it is assumed the further excellent fluidity of the spray powder mixture is assumed to contribute, in particular, to more uniform mixing with air in a powder/air mixing chamber mounted in a powder spraying apparatus, passage through a connection portion connecting the powder spraying apparatus main body and a spray nozzle, suppression of occurrence of clogging in the spray nozzle, and more uniform and stable spraying from the spray nozzle into a tooth surface above or below the gingival margin, or into a gingival sulcus and a periodontal pocket. The further excellent low-abrasive property of the spray powder mixture is assumed to contribute, in particular, to suppression of damage to a tooth surface by the spray powder mixture sprayed from a powder spraying apparatus to a tooth surface above or below the gingival margin.

[Background of the Invention]

The present inventors have extensively conducted studies on the reason why the spray powder mixtures of the conventional arts cannot be excellent in both fluidity and low-abrasive property. As a result, the following technical finding has been obtained. For example, spray powder including only the component (a) seems to have a reduced impact on a tooth surface simply because the component (a) itself is relatively soft. However, the component (a) is relatively soft, but tends to be easily aggregated, leading to an increase in particle diameter of particulate forms (for example, particles) in the spray powder. For this reason, the impact of the spray powder on the tooth surface increases, and consequently, damage to the tooth surface cannot be sufficiently suppressed. The increase in particle diameter also influences deterioration of fluidity, and consequently hamper, for example, mixing in a powder spraying apparatus and discharge from a spray nozzle. Thus, the present inventors have found the technical finding that the fundamental cause of being unable to be further excellent in fluidity and low-abrasive property lies in aggregability of particulate forms forming the spray powder.

The present inventors have further conducted studies on the basis of the above-described technical finding, and focused on the fact that the aggregability of the particles results from the hydrophilicity of the component (a). Thus, the present inventors have found that when hydrophobized silica fine particles (component (b)) as a second component are added to the component (a), the component (b) enters between the particles of the component (a), so that the aggregability of the spray powder mixture is significantly reduced. Thus, it has been found that the particle diameter of the component (a) is inhibited from considerably increasing in the spray powder mixture, so that deterioration of the low-abrasive property and fluidity are suppressed.

In this way, the present inventors have arrived at the spray powder mixture according to the present embodiment, which is characterized by comprising the specific component (a) (at least one selected from the group consisting of a saccharide, a sugar alcohol, an amino acid, a phosphate compound, a carbonate compound, and a calcium compound), and the specific component (b) (hydrophobized particulate silica).

[Findings in the Patent Documents]

The inventors of the present application have found out the following matters from patent documents. Spray powder mixtures and powder spraying apparatuses for preventing and treating periodontal disease have been studied. For example, a powder spraying apparatus capable of efficiently performing cleaning by spraying, together with water, a water-soluble abrading material mixed with air to a tooth surface above the gingival margin, and powder used for the apparatus are disclosed (Patent Document 1: US Patent No. 4174571 and Patent Document 2: US Patent No. 4595365). In this prior art, sodium hydrogen carbonate is used as a water-soluble abrading material that is mixed with air and sprayed to the tooth surface. The use of this abrading material has no problem in cleaning of the enamel of the tooth, but significantly damages dentin in which a tooth root portion is exposed, leading to a serious clinical problem. Therefore the range of use of the abrading material is limited. Patent Document 1 (US Patent No. 4174571) discloses, as an abrading material for a powder spraying apparatus, sodium glutamate or sodium gluconate which is less abrasive and less damaging to an enamel surface and a tooth root surface than abrading materials that have been heretofore used. Patent Document 3 (JP-A-4418113) discloses water-soluble dentin-friendly powder of any of amino acids, sugars, organic acids, salts thereof and the like in cleaning below the gingival margin such as cleaning of tooth root dentin, the powder having a density of 2.0 g/cm$^3$ or less and a mean particle size of 45 $\mu$m or less. Further, Patent Document 4 (US Patent No. 6126444) discloses that a fragile tooth root surface below the gingival margin is treated with a water-insoluble particulate polysaccharide, for example, powder of cellulose, starch, agar or the like. It is important that the powder does not remain in an oral cavity, particularly in a gingival sulcus and a periodontal pocket after being sprayed to the tooth

surface. However, if the powder remains in the oral cavity, not only a sense of discomfort is given to the patient, but also the powder remaining in the gingival sulcus, the periodontal pocket and the gingiva in the oral cavity may provide scaffolds, leading to induction of infection.

In such tooth surface cleaning of powder spraying type, powder particles having a large size give a large impact force to improve cleaning efficiency, but may increase damage to the cleaned tooth surface. On the other hand, if the powder particles have a small size, the impact force decreases, so that desired efficiency cannot be obtained. Thus, Patent Document 5 (US Patent No. 5810587) discloses a composition of powder in which groups of very small aluminum oxide particles in the range of 0.01 to 5 μm aggregate to form large particles having a size of 10 to 200 μm, and the particles are disrupted and loosened when collide with a tooth surface, so that the powder is satisfactory in both cleaning efficiency and damage to the tooth surface. Patent Document 6 (JP-A-5846720) discloses, as an abrasive for a tooth surface, powder containing alditol which is a sugar alcohol, the powder having a Mohs hardness of 4 or less and a mean particle size of 45 μm or less.

In the spray cleaning of a tooth surface with powder mixed with air in a powder/air mixing chamber of a powder spraying apparatus, it is important that plaque and calculus firmly stuck on a tooth surface are efficiently removed, and damage to the tooth surface is small. The powder should be dissolved in water and completely discharged outside the oral cavity after the spray cleaning. When particles having a large size are used as powder for spray cleaning, spray cleaning efficiency is enhanced, but damage to the tooth surface increases. For overcome this, two methods have been proposed. One method is using powder obtained by aggregating powder including small particles into large particles, and the other method is using, as powder, a water-soluble sugar alcohol or the like that is an organic substance which is a soft material and is hardly influenced by the size of particles. However, the surface area of powder further increases when the particle size of the powder decreases, or the powder itself has hydrophilicity because OH groups and the like having high affinity with water are present on the surface of powder of a water-soluble organic substance or the like, so that there is marked aggregability, resulting in deterioration of fluidity of the powder itself. In this case, a state of being uniformly mixed with air is not achieved in a powder/air mixing chamber mounted in a powder spraying apparatus, clogging occurs inside the powder/air mixing chamber mounted in the powder spraying apparatus, and in a connection portion through which the powder flows to a spray nozzle, the spray nozzle, and the like, and a problem may occur that spraying from the spray nozzle to the tooth surface is not uniform and stable.

Further, it is necessary to completely discharge the powder outside the oral cavity after the spray cleaning, and therefore the powder used is preferably water-soluble. However, since the inside of the gingival sulcus and the periodontal pocket is a narrow region, has little moisture and is in a wet state, there is a risk that the powder may remain in this site as a semi-dissolved material or an undissolved material, and provide habitats for bacteria, leading to induction of disease such as periodontal disease. Thus, a technique using highly water-soluble powder has been proposed. However, since a tooth surface in an oral cavity is in an environment at a temperature of about 37°C unlike a powder/air mixing chamber of a powder spraying apparatus, and the powder is mixed with water jetted through another path, and sprayed to the tooth surface together with the water during spray cleaning of the tooth surface, there is also a problem that the powder is already softened at the time when the powder is splayed to collide against the tooth surface, so that the spray cleaning efficiency decreases.

[Findings and the like Forming the Base of the Invention]

On the basis of the findings and the like derived from the prior arts, the present inventors have extensively conducted studies for solving the above-described problems, and resultantly found the following new findings (1) to (4).

(1) The fluidity of powder is known to be influenced by the shape of the powder itself, the particle size and the surface shape. However, in the present embodiment, it has been found as a first new finding that when as a second component, particulate silica subjected to specific hydrophobization treatment is mixed with powder having high hydrophilicity and high aggregability, the state of existence of each powder in the powder mixture changes, resulting in significant improvement of the fluidity of the powder mixture.

(2) The ability to remove calculus and plaque firmly stuck on a tooth surface and reduction of damage to the cleared tooth surface are trade-off characteristics. It is apparent from the conventional arts that when the powder has a large particle size and high hardness, and is water-insoluble, removal of calculus and plaque firmly stuck on a tooth surface is improved, but damage to the cleared tooth surface increases. There is a prior art in which an organic substance or the like that is a soft material is used for overcoming the above-mentioned problem, but there is a problem that the powder is poor in fluidity because of high hydrophilicity, and the powder has high aggregability and forms large secondary particles, and the above-described problem has not been overcome. However, it has been found as a second new finding that in the present embodiment, the spray powder mixture of item (1) maintains not only fluidity but also the trade-off characteristics, which are the problem in the present invention, in a well-balanced manner because the state of existence of each particle changes.

(3) Studies have been conducted focusing on the structure and operation of a powder spraying apparatus using the spray powder mixture according to the present embodiment, and as a result, the following has been revealed. Since the powder is sprayed together with water flowing out from another path in the process of spraying the powder from a nozzle tip portion, the powder is in a state of being mixed with water on a tooth surface. Thus, the ability to remove calculus and plaque firmly stuck on the tooth surface is also influenced by the solubility of the powder mixture in water. It has been found as a third new finding that rather than the problem of mere solubility such that powder is soluble or insoluble in water, the solubility is important because the intraoral temperature at the time when the powder arrives at the tooth surface also has an influence. That is, it is necessary that such a powder mixture exist in a powder state and engage in removal of calculus and plaque firmly stuck on the tooth surface immediately after spraying to the tooth surface, and the powder mixture be easily dissolved in water through gargling or the like and discharged outside the oral cavity after spray cleaning. If solubility in water is excessively high, the powder softens when sprayed to the tooth surface, so that it is not possible to achieve the original purpose of removing calculus or plaque firmly stuck on the tooth surface. On the other hand, if solubility in water is low, the powder remains on the periphery of the tooth surface and in the gingival sulcus and the periodontal pocket after spray cleaning, and provides habitats for bacteria and the like, leading to the possibility of inducing a disease.

(4) Further, after spray cleaning of the tooth surface or the inside of the gingival sulcus and periodontal pocket using a powder spraying apparatus, a semi-dissolved material or an undissolved material may remain even when gargling is performed because the inside of the oral cavity, particularly the inside of the gingival sulcus and periodontal pocket is a narrow region, has little moisture, and is in a wet state. As described above, the powder is water-soluble, and therefore may stick to various intraoral sites as an undissolved material or a semi-dissolved material in the oral cavity in a wet environment with little water, and the powder can also provide scaffolds for bacterial infection, so that intraoral disease may be induced. Thus, in the present embodiment, it has been found as a fourth new finding that when the powder mixture comprises ion-sustained-release glass as a third component, it is possible to prevent the onset of intraoral disease by sustained release of various ions. This enables suppression of adhesion and growth of bacteria even if the powder mixture remains in the oral cavity in a semi-dissolved or undissolved state without being completely dissolved in water, and further, it is possible to expect suppression of dental caries and periodontal disease due to the effect of strengthening the tooth substance of the tooth surface and functioning as a barrier for the soft tissue in the periodontal pocket by the effect of sustained release of ions as mentioned above.

[Embodiments and the like derived on the Basis of New Findings and the like]

The present inventors have clarified a plurality of embodiments of the present invention and their effects on the basis of the above-described new findings and the like. For example, it is preferable that the spray powder mixture according to the present embodiment comprises (c) ion-sustained-release glass in addition to the components (a) and (b). It has been revealed that in the spray powder mixture according to the present embodiment, mixing of the component (a) and the component (b) creates a state in which the periphery the component (a) as large particles is coated with hydrophobized small particles as the component (b), so that the fluidity of the powder mixture is improved. When a sugar alcohol as the component (a) and trialkylsilylated particulate silica as the component (b) are mixed, the effect is particularly pronounced due to interaction between the particle surfaces.

It has been revealed that the improvement of fluidity enables attainment of both ability to remove calculus and plaque firmly stuck on the tooth surface and reduction of damage to the cleared tooth surface, which are trade-off characteristics. When a sugar alcohol, preferably a sugar alcohol obtained by reducing a disaccharide, more preferably a sugar alcohol whose solubility in water at 20°C is 30 wt% or less is used as the component (a), the effect is particularly pronounced.

The component (a) comprised in the powder mixture is preferably water-soluble for maintaining the trade-off characteristics, but even when gargling is performed after the powder is sprayed to the tooth surface using a powder spraying apparatus, a semi-dissolved material or an undissolved material may remain at the site because the inside of the oral cavity, particularly the inside of the gingival sulcus and periodontal pocket is a narrow region, has little moisture, and is in a wet state. The undissolved material or semi-dissolved material remaining in the oral cavity may provide habitats for bacteria, leading to induction of disease such as periodontal disease. Therefore, in the present embodiment, the powder mixture contains ion-sustained-release glass as the component (c), and at least one type of ions among various ions, particularly fluorine ions, strontium ions, borate ions, and aluminum ions are released in a sustained manner. Thus, it can be expected that even if an undissolved material or a semi-dissolved material remains in the oral cavity, those ions are continually released in a sustained manner, so that adhesion and growth of bacteria can be suppressed, and further, strengthening of the tooth substance of the tooth surface, a function as a barrier for the soft tissue in the periodontal pocket during powder spray cleaning, and the like are exhibited, thereby helping suppress the onset of dental caries and periodontal disease.

Hereinafter, the component (a), the component (b) and optional components including the component (c) in

the spray powder mixture according to the present embodiment will be each described in detail.

[Component (a)]

The spray powder mixture according to the present embodiment comprises the component (a). The component (a) can efficiently remove plaque and calculus firmly stuck on the tooth surface while reducing damage to the tooth surface (particularly dentin) when the spray powder mixture is sprayed to the tooth surface by a spraying apparatus.

The component (a) is at least one selected from the group consisting of a saccharide, a sugar alcohol, an amino acid, a phosphate compound, a carbonate compound, and a calcium compound. The component (a) may be a mixture thereof. Among these components (a), a saccharide, a sugar alcohol, and a carbonate compound are preferable, and a sugar alcohol is more preferable from the viewpoint of placing importance on further improvement of the fluidity and the low-abrasive property of the spray powder mixture. Among the sugar alcohols, a sugar alcohol that is a reduced disaccharide is still more preferable, and from the viewpoint of placing importance on improvement of the removal ability of the spray powder mixture, a sugar alcohol whose solubility in water at 20°C is 30 wt% or less is particularly preferable.

If the solubility of the sugar alcohol is 30 wt% or less at 20°C, the solubility of the sugar alcohol in water is relatively low. Thus, when the spray powder mixture is sprayed by a powder spraying apparatus at ambient temperature (about 20°C), the sugar alcohol comprised in the spray powder mixture is hardly dissolved in the water sprayed, and can collide against the tooth surface in the form of powder (solid). Therefore, in such a case, the spray powder mixture is excellent in removal ability, and plaque and calculus firmly stuck on the tooth surface can be efficiently removed.

(Saccharide)

Any saccharide can be used regardless of the shape of powder as long as the saccharide is in a powder form at ambient temperature. Examples of the saccharide comprise monosaccharides, disaccharides, trisaccharides, and polysaccharides, and mixtures thereof can be used without limitation. Examples of the monosaccharide comprise monosaccharides called aldose which have one aldehyde group (-CHO) at a terminal in a molecule of chain structure, monosaccharides called ketose which have one ketone group (= CO) in a molecule of chain structure, and monosaccharides which are deoxy sugars in which one of hydroxy groups in the molecule is reduced into a hydrogen atom.

Examples of the monosaccharide called aldose which have one aldehyde group (-CHO) at a terminal in a molecule of chain structure comprise, but are not limited to, glyceraldehyde, arabinose, lyxose, allose, altrose, glucose, mannose and the like. Examples of the monosaccharide called ketose which have one ketone group (=CO) at a terminal in a molecule of chain structure comprise, but are not limited to, dihydroxyacetone, psicose, fructose, sorbose, tagatose and the like. Examples of the monosaccharide which is a deoxy sugar in which one of hydroxy groups in the molecule is reduced into a hydrogen atom include, but are not limited to, deoxyribose, fucose, rhamnose and the like.

Examples of the disaccharide comprise, but are not limited to, sucrose, lactose, maltose, trehalose, turanose, cellobiose, palatinose and the like. Among such disaccharides, mention is made of, for example, lactose, maltose, trehalose, turanose, cellobiose palatinose and the like, preferably trehalose and palatinose, from the viewpoint of placing importance on suppression of the onset of dental caries.

Further, any oligosaccharide can be used without any limitation for trisaccharides and polysaccharides (more specifically, trisaccharides and higher oligosaccharides). In addition, some of the saccharides described above have asymmetric carbon in the molecule and have stereoisomers, but all of them are included under the names written herein.

Among such saccharides, mention is made of naturally abundant saccharides or industrially producible saccharides from the viewpoint of placing importance on cost reduction, and mention may be made of, for example, trehalose, fructose, tagatose, maltitose and palatinose (isomaltulose) and the like which are generally applied to food, from the viewpoint of placing importance on the dental caries onset inhibitory property (resistance to onset of caries) in addition to the viewpoint of placing importance on cost reduction.

(Sugar Alcohol)

Any sugar alcohol can be used regardless of the shape of powder as long as the sugar alcohol is in a powder (solid) form. Examples of the sugar alcohol comprise monosaccharides and disaccharides, and mixtures thereof can be used without limitation. Among such sugar alcohols, the monosaccharide is, for example, a sugar alcohol produced by reducing the carbonyl group of the monosaccharide aldose or ketose described in the saccharides above. Examples of the monosaccharide sugar alcohol called alditol (sugar alcohol obtained by reducing aldose as a saccharide having an aldehyde group: -CHO in a molecule of chain structure to convert the aldehyde group into a hydromethyl group) comprise, but are not limited to, erythritol, threitol, ribitol, xylitol, arabinitol, glucitol (sorbitol), mannitol and the like. Examples of the disaccharide sugar alcohol (that is, sugar alcohol obtained by reducing a disaccharide as a saccharide) comprise, but are not limited to, lactitol, maltitol, reduced palatinose and the like. In addition, some of the sugar alcohol described

above have asymmetric carbon in the molecule and have stereoisomers, but all of them are included under the names written herein. Among such sugar alcohols, mention is made of, for example, naturally abundant sugar alcohols or industrially producible sugar alcohols from the viewpoint of placing importance on cost reduction, mention may be made of, for example, erythritol, xylitol, glucitol (sorbitol), mannitol, maltitol, reduced palatinose and the like, with erythritol, mannitol and reduced palatinose being preferable, from the viewpoint of placing importance on resistance to induction of dental caries in addition to the viewpoint of placing importance on cost reduction. Among such sugar alcohols, erythritol, xylitol, maltitol and reduced palatinose which are approved as special health food are more preferable. Further, reduced palatinose is also called isomalt, an isomaltulose reduction product or palatinite as another name, and is the most preferred sugar alcohol because palatinose as a precursor has a reduction group in the fructose moiety in the molecule, and the $\alpha$-1,6-glucoside bond is stable enough to complete the reaction while maintaining the fundamental molecular structure of the disaccharide without causing hydrolysis under hydrogenation conditions, so that the final product is an equimolar mixture of $\alpha$-D-glucopyranosyl-1,6-mannitol and $\alpha$-D-glucopyranosyl-1-6-sorbitol as a stereoisomer thereof, which has properties intermediate between both the compounds.

(Amino Acid)

Any amino acid can be used regardless of the shape of powder as long as the amino acid is in a powder (solid) form. Examples of the amino acid comprise acidic amino acids having two carboxyl groups in the molecule, basic amino acids having two or more amino groups in the molecule, and neutral amino acids having characteristic groups other than a carboxyl group and an amino group (more specifically, a hydroxyl group, an amide group and an aromatic group), a characteristic site (more specifically, an alkyl chain or the like), or a characteristic atom (more specifically, a sulfur atom) in the molecule, and mixtures thereof can be used without limitation. Examples of the amino acid are as follows. Examples of the acidic amino acid having two carboxyl groups in the molecule comprise aspartic acid and glutamic acid. Examples of the basic amino acid having two or more amino groups in the molecule comprise lysine, arginine and histidine. Examples of the neutral amino acid having an alkyl chain in the molecule comprise glycine, alanine, valine, leucine and isoleucine. Examples of the neutral amino acid having a hydroxy group in the molecule comprise serine and threonine. Examples of the neutral amino acid comprising a sulfur atom in the molecule comprise cysteine and methionine. Examples of the neutral amino acid having an amide group in the molecule comprise asparagine and glutamine. Examples of the neutral amino acid having an imino group in the molecule include proline. Examples of the amino acid having an aromatic group in the molecule comprise, but are not limited to, phenylalanine, tyrosine, tryptophan and the like. Some of the amino acids described above have asymmetric carbon in the molecule and have stereoisomers, but all of them are included under the names written herein. Among such amino acids, mention may be made of, for example, histidine, tryptophan, lysine, methionine, phenylalanine, threonine, valine, leucine, isoleucine classified as essential amino acids which are amino acids that cannot be synthesized in the body of animal, and glycine that is abundantly comprised in collagen which is an animal protein and the like.

(Phosphate Compound)

Any phosphate compound can be used regardless of the shape thereof as long as the phosphate compound is in a powder (solid) form. Examples of the phosphate compound comprise tricalcium phosphate, calcium dihydrogen phosphate, calcium monohydrogen phosphate, calcium hydroxyphosphate (synthetic hydroxyapatite) and the like, and mixtures thereof can be used without any problem, but the phosphate compound is not limited thereto.

Among such phosphate compounds, mention may be made of, for example, calcium hydroxyphosphate (synthetic hydroxyapatite) comprising the same component as natural hydroxyapatite contained in enamel or dentin of the tooth.

(Carbonate Compound)

Any carbonate compound can be used regardless of the shape thereof as long as the carbonate compound is in a powder (solid) form. Examples of the carbonate compound comprise heavy calcium carbonate (natural calcium carbonate) obtained by grinding and/or classifying limestone which is calcium carbonate, light calcium carbonate (more specifically, synthetic calcium carbonate, precipitated calcium carbonate and the like) obtained by precipitating fine crystals in a liquid by a chemical reaction, calcium hydrogen carbonate, sodium hydrogen carbonate (sodium bicarbonate), sodium carbonate and the like, and mixtures thereof can be used without limitation, but the carbonate compound is not limited thereto. Among such carbonate compounds, mention may be made of, for example, sodium hydrogen carbonate (sodium bicarbonate) having a usage record as a powder raw material with conventional powder spraying apparatuses and the like.

(Calcium Compound)

Any calcium compound can be used regardless of the shape thereof as long as the calcium compound is in a powder (solid) form. Examples of the calcium compound include calcium oxide, calcium hydroxide (slaked lime), calcium fluoride and the like, but are not limited thereto.

Among them, calcium fluoride or the like is preferably used which is capable of strengthening the tooth by fluorinating natural hydroxyapatite comprised in enamel or dentin of the tooth.

The components (a) can be classified into organic powder and inorganic powder, the former corresponds to a saccharide, a sugar alcohol, an amino acid or the like, and the latter corresponds to a phosphate compound, a carbonate compound, a calcium compound or the like. Since these classified powders are different in hardness of the material, the impact force in removal of calculus and plaque firmly stuck on the tooth surface varies even if the particle diameters are the same, and as a result, the ability to remove calculus and plaque firmly stuck on the tooth surface, damage to the tooth surface, and the like may be influenced. Thus, from the viewpoint of placing importance on improvement of the removal ability and the low-abrasive property of the spray powder mixture, an appropriate particle diameter can be selected according to a type of the classified powder. More specifically, since organic powder such as powder of a saccharide, a sugar alcohol or an amino acid is a soft material having a hardness lower than that of inorganic powder, the mean particle diameter (D50) of the organic powder may be, for example, 0.1 to 200.0 $\mu$m, and is preferably 1.0 to 100.0 $\mu$m, more preferably 5.0 to 80.0 $\mu$m, still more preferably 10.0 to 50.0 $\mu$m, particularly preferably 20.0 to 35.0 $\mu$m. On the other hand, since inorganic powder such as powder of a phosphate compound, a carbonate compound, or a calcium compound is a material having a hardness higher than that of organic powder, the mean particle diameter (D50) of the inorganic powder may be, for example, 0.1 to 100.0 $\mu$m, and is preferably 1.0 to 80.0 $\mu$m, more preferably 5.0 to 70.0 $\mu$m, still more preferably 10.0 to 70.0 $\mu$m, particularly preferably 30.0 to 70.0 $\mu$m.

In the present specification, the "mean particle diameter (D50)" can be measured using a laser diffraction/scattering method. That is, the mean particle diameter (D50) is a 50% cumulative particle diameter value on the smaller diameter side in a particle size distribution obtained by the laser diffraction/scattering method. However, the component (a) comprised in the spray powder mixture according to the present embodiment is often water-soluble, determination of the mean particle diameter (D50) by a dry particle size measurement method is more appropriate.

If the mean particle diameter (D50) is below the above-described range, the fluidity of the spray powder mixture according to the present embodiment is impaired, so that a state of being uniformly mixed with air is not achieved in a powder/air mixing chamber mounted in a powder spraying apparatus, clogging occurs inside the powder/air mixing chamber mounted in the powder spraying apparatus, and in a connection portion through which the powder flows to a spray nozzle, the spray nozzle, and the like, and a problem may occur that spraying from the spray nozzle to the tooth surface is not uniform and stable. Further, if the particle diameter is small, satisfactory results cannot be obtained in the ability to remove calculus and plaque firmly stuck on the tooth surface because the impact force decrease. On the other hand, it is not preferable that the average particle diameter (D50) exceeds the above-described range because the particle diameter increases, and accordingly, the impact force increases, so that the tooth surface after removal of the stuck calculus and plaque is damaged.

In a preferred aspect, the spray powder mixture according to the present embodiment comprises, as the component (a), organic powder such as powder of a saccharide, a sugar alcohol or an amino acid, which is hardly influenced by the hardness of the material and can be controlled only by the mean particle diameter (D50), for well-balanced maintenance of the ability to remove calculus and plaque firmly stuck on the tooth surface and the degree of damage to the tooth surface after spray cleaning.

Further, the tooth surface from which plaque and calculus are removed has moisture and is hydrophilic, whereas in the spray powder mixture according to the present embodiment, the entire spray powder mixture is made hydrophobic by mixing hydrophobized particulate silica as the component (b) for imparting fluidity. Thus, the spray powder mixture has a property of being hardly compatible with the tooth surface to which the powder mixture is sprayed, and the ability to remove plaque and calculus firmly stuck on the tooth surface largely depends on just the impact force during spraying of the powder. However, it has been revealed that, in the spray powder mixture according to the present embodiment, a hydrophilic material having high hydrophilicity is used as parent particles as the component (a), and thus collision and adhesion with the hydrophilic tooth surface occur at the same time during spray cleaning, so that it is possible to efficiently remove calculus and plaque firmly stuck on the tooth surface, and it is also possible to reduce damage to the tooth surface. Thus, in a preferred aspect, the spray powder mixture according to the present embodiment comprises a hydrophilic sugar alcohol as the component (a) among organic powders. Examples of the hydrophilic sugar alcohol comprise sugar alcohols having a large number of OH groups having high affinity with water in the molecule, which can be obtained by reducing carbonyl groups of a saccharide into hydrogen.

In the spray powder mixture according to the present embodiment, it is preferable that the component (a) is hydrophilic as described above. In such a case, from the viewpoint of placing importance on suppression of periodontal disease, it is preferable that the component (a) has hydrophilicity that is not either excessively high or excessively low,

but is moderate. When the component (a) has hydrophilicity (water solubility) that is not excessively high, the component (a) in the spray powder mixture sprayed to the tooth surface is hardly dissolved in water even in an oral cavity in an environment at about 37°C (different from the inside of the powder spraying apparatus) because the powder mixture is sprayed to the tooth surface together with water flowing out from another path of the powder spraying apparatus. For this reason, the component (a) tends to hardly soften, so that it is possible to suppress deterioration of ability to remove calculus and plaque firmly stuck on the tooth surface. On the other hand, it is necessary to completely discharge the spray residue outside the oral cavity after the spray cleaning. If the solubility of the powder sprayed is excessively low, a part of the powder is incompletely dissolved, and therefore there is a possibility the semi-dissolved material sticks/remains in the oral cavity, and the residue cannot be discharged outside the oral cavity, thus providing scaffolds for adhesion of bacteria, leading to periodontal disease.

Thus, it has been revealed that even a sugar alcohol having affinity for water influences the effects of the present invention depending on a type or a molecular structure thereof, and a solubility based on the molecular structure. Examples of the solubility of the sugar alcohol in water (the amount of the sugar alcohol dissolved in 100 mL of water at 20°C) are as follows: sorbitol: 72 wt%, xylitol: 66 wt%, mannitol: 18 wt% and erythritol: 33 wt% for monosaccharide sugar alcohols; and maltitol: 60 wt% and reduced palatinose: 28 wt% for disaccharide sugar alcohols. Thus, in the present embodiment, use of a sugar alcohol is a preferred aspect, and for more pronouncedly exhibiting the effects of the present invention, a sugar alcohol having a disaccharide structure is more preferable than a monosaccharide sugar alcohol having an excessively high solubility in water, and the solubility in water (solubility in 100 mL of water at 20°C) is more preferably 30 wt% or less. Further, regarding the solubility in water, an excessively low solubility in water may cause remaining after spray cleaning, and therefore in the most preferred aspect, the solubility in water is in the range of 20 to 30 wt%, from the viewpoint that the sugar alcohol can be dissolved in the oral cavity and completely discharged outside the oral cavity.

The content of the component (a) when the total amount of the spray powder mixture according to the present embodiment is 100 parts by weight may be, for example, 80 parts by weight or more, preferably 90 parts by weight or more, more preferably 95 parts by weight or more.

[Component (b)]

The component (b) imparts hydrophobicity to the spray powder mixture. The hydrophobized particulate silica as the component (b) can be used without limitation as long as it is hydrophobized by subjecting fine silica particles having primary particles of 0.01 to 1000 nm to surface treatment with an organic compound in a state of primary particles or in a state of being processed into aggregated particles or agglomerated particles. The primary particles of silica may be, for example, 0.01 to 1000 nm, 1 to 100 nm or 5 to 20 nm. The method for producing particulate silica is not particularly limited. Particulate silica produced by any production method such as a dry method (high-temperature hydrolysis method) for producing dry silica using silicon tetrachloride or the like as a raw material, a wet method for producing precipitated silica using water glass or the like as a raw material, or sol-gel silica for producing sol-gel silica using an alkoxide compound or the like as a raw material can be used, and mixed particulate silica obtained by mixing silicas produced by different methods is not limited. Further, the crystallinity is not particularly limited, and particulate silica that is crystalline or non-crystalline, and any mixture thereof are acceptable. Among them, non-crystalline particulate silica produced by a dry method and obtained by processing fine silica particles having primary particles of 1 to 100 nm into aggregated particles or agglomerated particles is preferably used. When measured by a BET method, the specific surface area of the particulate silica formed into aggregated particles or agglomerated particles by the above-mentioned processing may be, for example, 20 to 400 ($m^2/g$), and is preferably 50 to 300 ($m^2/g$), more preferably 100 to 300 ($m^2/g$). These particulate silicas can be used alone, or in combination of two or more thereof.

While it is an important requirement in the present embodiment to hydrophobize the above-described particulate silica by performing surface treatment with an organic compound, any organic compound can be used without limitation as long as it can be hydrophobized. By treating the surface of the particulate silica with an organic compound, the property of the surface of the particulate silica can be changed from a hydrophilic surface into a hydrophobic surface, but the degree of hydrophobization varies depending on conditions such as a surface treatment method and an amount of surface treatment with the organic compound. Thus, for definition of hydrophobization mentioned here, particulate silica subjected to hydrophobization treatment is considered to have been hydrophobized in the present embodiment if the particulate silica at least slightly floats on the surface of water when the particulate silica is added to the water. As the organic compound that can be used for the hydrophobization treatment, any organic compound can be used without limitation, use of a silane compound capable of being subjected to condensation reaction with OH groups on the surface of the particulate silica is a preferred aspect.

Examples of the hydrophobization treatment include a hydrophobization treatment by dimethylsilylation, trimethylsilylation, alkylsilylation, trialkylsilylation, dimethylpolysiloxane, aminoalkylsilylation, methacrylsilylation, methacrylalkylsilylation and the like. Among them, hydrophobization treatment by dialkylsilylation or trialkylsilylation is preferable,

hydrophobization treatment by dimethylsilylation or trimethylsilylation is more preferable, and hydrophobization treatment by trimethylsilylation is still more preferable. Examples of the silane compound that can be used for hydrophobization treatment comprise dimethyldichlorosilane, trimethylchlorosilane, hexamethyldisilazane and the like. These hydrophobized particulate silicas can be used alone, or in combination of two or more thereof. Further, hydrophobized particulate silica granulated or aggregated to have a larger particle diameter can be used without limitation.

Particulate titania, particulate alumina and other oxide fine particles produced by the same production method as that for the particulate silica used in the present embodiment and having a similar particle diameter and specific surface area can also be used in the present embodiment when subjected to hydrophobization treatment. Further, particles obtained by mixing these particulate oxides and the above-described particulate silica and subjecting the mixture to hydrophobization treatment, or particles obtained by mixing respective fine particles subjected to hydrophobization treatment can also be used as the spray powder mixture according to the present embodiment.

The content (mixing ratio) of the hydrophobized particulate silica as the component (b) is not particularly limited, and the hydrophobized particulate silica can be mixed in any proportion. The content of the hydrophobized particulate silica as the component (b) may be, for example, 0.001 parts by weight to 10.0 parts by weight, preferably 0.01 parts by weight to 8.0 parts by weight, more preferably 0.1 parts by weight to 5.0 parts by weight, per 100 parts by weight of the component (a). When the content of the hydrophobized particulate silica per 100 parts by weight of the component (a) is 0.001 parts by weight or more, the fluidity of the spray powder mixture is improved. That is, a state in which the spray powder mixture is uniformly mixed with air is easily achieved in a powder/air mixing chamber mounted in a powder spraying apparatus, clogging is unlikely to occur inside the powder/air mixing chamber mounted in the powder spraying apparatus, and in a connection portion through which the powder flows to a spray nozzle, the spray nozzle, and the like, and a problem may occur that spraying from the spray nozzle to the tooth surface is not uniform and stable. On the other hand, when the content of the hydrophobized particulate silica per 100 parts by weight of the component (a) is 10.0 parts by weight or less, it is possible to suppress deterioration of workability while maintaining the effects of the present invention. Specifically, in such a case, the hydrophobized particulate silica does not exist in an excessive amount in the spray powder mixture, and therefore the bulk of the spray powder mixture is unlikely to increase, so that there is not difficulty in handling such that filling operations during transfer into the powder/air mixing chamber are difficult to carry out.

[Component (c)]

(c) Ion-sustained-release glass continually releases ion species in a sustained manner on the basis of a glass composition. Preferably, the spray powder mixture according to the present embodiment further comprises (c) ion-sustained-release glass. When the spray powder mixture further comprises (c) ion-sustained-release glass, the (c) ion-sustained-release glass imparts an ion species-sustained-release property to the spray powder mixture. Therefore, in such a case, it is possible to exhibit strengthening of the tooth, suppression of the activity of bacteria, and the like for tooth above and below the gingival margin, the soft tissues in the gingival sulcus and the periodontal pocket, and the like by the effect of sustained release of ions.

Preferably, the (c) ion-sustained-release glass is capable of releasing at least any one of a fluorine ion, a strontium ion, a borate ion, and an aluminum ion in a sustained manner. When the (c) ion-sustained-release glass performs sustained-release of any one of the above-described ions, it is possible to further exhibit strengthening of the tooth, suppression of the activity of bacteria, and the like. In a more preferred aspect, in the (c) ion-sustained-release glass, the plurality of ion species are simultaneously released in a sustained manner.

Any ion-sustained-release glass can be used without limitation as long as the ion-sustained-release glass contains one or more glass framework forming elements that form a glass framework and one or more glass modifying elements that modify the glass framework. These ion-sustained-release glasses can be used alone, or in combination of two or more thereof. In the present embodiment, glass double-function elements having the roles of both a glass framework forming element and a glass modifying element depending on a glass composition is included as a category of glass framework forming elements. Examples of the glass framework forming element comprised in the ion-sustained-release glass comprise silica, aluminum, boron, phosphorus and the like, and these elements can be used alone, or in combination of two or more thereof. Examples of the glass modifying element comprise halogen elements such as fluorine, bromine, iodine and the like, alkali metal elements such as sodium, lithium and the like, and alkaline earth metal elements such as calcium, strontium and the like, and these elements can be used alone or in combination of two or more thereof. It is preferable that among them, silica, aluminum and boron are comprised as glass framework forming elements, and fluorine, sodium and strontium are comprised as glass modifying elements, and examples include silica glass containing strontium and sodium, fluoroaluminosilicate glass, fluoroborosilicate glass, fluoroaluminoborosilicate glass and the liek. Further, from the viewpoint of sustained release of fluorine ions, strontium ions, borate ions, and aluminum ions, mention may be made of, for example, fluoroaluminoborosilicate glass containing strontium. Examples of the glass composition range are as follows: $SiO_2$ at 15 to 35 mass%, $Al_2O_3$ at 15 to 30 mass%, $B_2O_3$ at 5 to 20 mass%, SrO at 20 to 45 mass%, F at 5 to 15 mass%, and $Na_2O$ at 0 to 10 mass%. This glass composition can be

confirmed by instrumental analysis such as elemental analysis, Raman spectrum, X-ray fluorescence analyses and the like, and any analysis method is acceptable as long as the measured values match these composition ranges.

The method for producing such ion-sustained-release glass is not particularly limited, and the ion-sustained-release glass can be produced by a production method such as a melting method or a sol-gel method. Among them, a method of production by a melting method using a melting furnace is preferable from the viewpoint of ease of glass composition design including selection of raw materials. The ion-sustained-release glass used for the spray powder mixture according to the present embodiment has an amorphous structure, one having a crystalline structure in part is acceptable, and a mixture of glass having an amorphous structure and glass having a crystal structure is acceptable. Whether or not the glass structure is amorphous can be determined by X-ray diffraction analysis or use of an analytical instrument such as a transmission electron microscope. In particular, it is preferable that the ion-sustained-release glass for use in the present embodiment has an amorphous structure which is a heterogenous structure because various ions are released in a sustained manner depending on an equilibrium relationship with an ion concentration in an external environment.

The mean particle diameter (D50) of the ion-sustained-release glass influences fluidity in the spray powder mixture according to the present embodiment, the ability to remove calculus and plaque firmly stuck on the tooth surface, the degree of damage to the cleared tooth surface, and ion-sustained-releasability in which strengthening of the tooth surface, suppression of the activity of bacteria in the periodontal pocket, and the like are exhibited. As the "mean particle diameter (D50)" in the present specification, a mean particle diameter (D50) obtained by any particle size measurement method such as a laser diffraction/scattering method, a dynamic light scattering method, or a centrifugal sedimentation method or the like can be used without particular limitation regardless of the measurement principle. That is, in any of the particle size measurement methods, the mean particle diameter (D50) is a 50% cumulative particle diameter value on the smaller diameter side in a particle size distribution determined. The mean particle diameter (D50) of the ion-sustained-release glass can be controlled in production steps such as wet or/and dry grinding, classification, sieving and the like. The mean particle diameter (D50) of the ion-sustained-release glass may be, for example, 0.01 to 50.0 $\mu$, and is preferably 0.1 to 10.0 $\mu$m, more preferably 0.5 to 5.0 $\mu$m. When the mean particle diameter (D50) of the ion-sustained- release glass is 0.01 $\mu$m or more, the specific surface area of the ion-sustained-release glass decreases and thus the amount of sustained release of ions decreases, but it is possible to suppress deterioration of the fluidity of the spray powder mixture according to the present embodiment. As a result, a state of being uniformly mixed with air is easily achieved in a powder/air mixing chamber mounted in a powder spraying apparatus, clogging is unlikely to occur inside the powder/air mixing chamber mounted in the powder spraying apparatus, and in a connection portion through which the powder flows to a spray nozzle, the spray nozzle, and the like, and a problem is unlikely to occur that spraying from the spray nozzle to the tooth surface is not uniform and stable. On the other hand, when the mean particle diameter (D50) of the ion-sustained-release glass is 50.0 $\mu$m or less, the ability to remove calculus and plaque firmly stuck on the tooth surface when the spray powder mixture according to the present embodiment is sprayed to the tooth surface is deteriorated, but damage to the cleared tooth surface can be reduced.

The shape of the ion-sustained-release glass may be any shape such as a spherical shape, a plate shape, a fragmental shape and a scaly shape, and is not particularly limited, and a spherical shape and a fragmental shape are preferable.

The content of the (c) ion-sustained-release glass is not particularly limited, and the ion-sustained-release glass can be mixed in any proportion. For example, the content thereof may be 0.01 parts by weight to 30.0 parts by weight, and is preferably 0.1 parts by weight to 20.0 parts by weight, more preferably 0.5 parts by weight to 10.0 parts by weight, still more preferably 1.0 part by weight to 10.0 parts by weight, per 100 parts by weight of the component (a). When the content of the ion-sustained-release glass per 100 parts by weight of the component (a) is 0.01 parts by weight or more, an extreme decrease in the amount of sustained release of ions is suppressed, and it is possible to suppress adhesion and growth of bacteria even if the spray powder mixture according to the present embodiment remains in an undissolved or semi-dissolved state in the oral cavity, it is also possible to expect strengthening of the tooth surface and exhibition of a function as a barrier for the soft tissue in the periodontal pocket during spraying of the powder, and the onset of dental caries and periodontal diseases can be suppressed. On the other hand, when the content of the ion-sustained-release glass is 30.0 parts by weight or less, the ability to remove calculus and plaque firmly stuck on the tooth surface when the spray powder mixture according to the present embodiment is sprayed to the tooth surface is not significantly improved, but the risk of increasing damage to the cleared tooth surface decreases. The fluidity of the spray powder mixture according to the present embodiment is unlikely to be impaired, so that a state of being uniformly mixed with air is easily achieved in a powder/air mixing chamber mounted in a powder spraying apparatus, occurrence of clogging inside the powder/air mixing chamber mounted in the powder spraying apparatus, and in a connection portion through which the powder flows to a spray nozzle, the spray nozzle, and the like is suppressed, and the risk of occurrence of a problem spraying from the spray nozzle to the tooth surface is not uniform and stable decreases.

In a preferred aspect, for example, the ion-sustained-release glass may be surface-treated from the viewpoint of placing importance on further enhancement of ion-sustained-releasability from the ion-sustained-release glass as the

component (c). By treating the surface of the ion-sustained-release glass, the ion-sustained-release glass can be functionalized to improve ion-sustained-releasability. Examples of the surface treatment material used for the surface treatment comprise surfactants, fatty acids, organic acids, inorganic acids, monomers, polymers, various coupling materials, silane compounds, metal alkoxide compounds, partial condensates thereof and the like. These surface treatment materials may be used alone, or may be used in combination of two or more thereof. Among these surface treatment materials, acidic polymers and silane compounds are preferably used to perform composite surface treatment.

The composite surface treatment is a method in which the surface of ion-sustained-release glass is covered with a silane compound, and then subjected to surface treatment using an acidic polymer, which will be described in detail below.

In an aqueous dispersion comprising ion-sustained-release glass pulverized to a desired mean particle diameter (D50) by grinding or the like, a silane compound represented by general formula (I):

[General Formula]

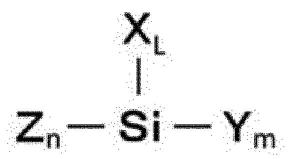

(I)

(wherein Z represents RO-, X represents a halogen, Y represents -OH, R represents an organic group having 8 or less carbon atoms, and n, m and L each represent an integer of 0 to 4, where n + m + L = 4) is mixed, the silane compound is hydrolyzed or partially hydrolyzed in the system to obtain a silanol compound, the silanol compound is then condensed to obtain a polysiloxane, and the surface of the ion-sustained-release glass is covered with the polysiloxane to obtain polysiloxane-covered ion-sustained-release glass.

Examples of the organic group having 8 or less carbon atoms and represented by R in the general formula (I) comprise an alkyl group having 1 to 8 carbon atoms, an alkenyl group having 2 to 8 carbon atoms, and a phenyl group. The alkyl group having 1 to 8 carbon atoms is linear or branched, and examples thereof comprise a methyl group, an ethyl group, a n-propyl group, an isopropyl group, a n-butyl group, a s-butyl group, a t-butyl group, a pentyl group, an isopentyl group, a neopentyl group, a n-hexyl group, a n-heptyl group and a n-octyl group. The alkyl group having 1 to 8 carbon atoms is preferably an alkyl group having 1 to 4 carbon atoms (more specifically, a methyl group, an ethyl group, a propoxy group or a butyl group), more preferably an alkyl group having 1 to 2 carbon atoms (more specifically, a methyl group or an ethyl group), still more preferably a methyl group. The alkenyl group having 2 to 8 carbon atoms is linear or branched, and examples thereof comprise an ethenyl group (vinyl group), a propenyl group, a butenyl group, a pentenyl group, a hexenyl group, a heptenyl group and an octenyl group.

Examples of the halogen represented by X in the general formula (I) comprise fluorine, chlorine, bromine and iodine. The halogen is preferably chlorine.

In the general formula (1), n representing the number of Z in one molecule of the silane compound represents preferably an integer of 1 to 4, more preferably an integer of 2 to 4, still more preferably an integer of 3 to 4, particularly preferably 4. The larger the value of n, the larger the number of RO groups in the silane compound represented by the general formula (1). The particulate silica subjected to hydrophobization treatment with the silane compound having a large value of n and represented by the general formula (1) has high hydrophobicity.

Examples of the silane compound represented by the general formula (I) comprise tetramethoxysilane, tetraethoxysilane, tetrapropoxysilane, tetraallyloxysilane, tetrabutoxysilane, tetrakis(2-ethylhexyloxy)silane, trimethoxychlorosilane, triethoxychlorosilane, triisopropoxychlorosilane, trimethoxyhydroxysilane, diethoxydichlorosilane, tetraphenoxysilane, tetrachlorosilane and silicon hydroxide (silicon oxide hydrate) and the like, and tetramethoxysilane and tetraethoxysilane are preferable.

A low-condensation product of the silane compound represented by the general formula (I) is more preferable. For example, it is a low-condensation silane compound obtained by condensing tetramethoxysilane and tetraethoxysilane by partial hydrolysis thereof. These compounds can be used alone or in combination.

An organosilane compound can also be added as a part of the silane compound represented by the general formula (I) during the polysiloxane treatment.

By subjecting the polysiloxane-covered ion-sustained-release glass obtained in the previous step to acidic polymer treatment in which the ion-sustained-release glass is reacted with an acidic polymer, ion-sustained-release glass which is a particularly preferred component (c) to be mixed with the spray powder mixture according to the present embodiment. For the acidic polymer treatment, equipment generally used in the industry can be used as long as it is a dry flow-type stirrer, and examples thereof comprise a Hensil mixer, a super mixer, a high speed mixer and the like. The

reaction of the acidic polymer with the ion-sustained-release glass on which a polysiloxane film is formed can be carried out by bringing an acidic polymer solution into contact with the ion-sustained-release glass by impregnation, spraying or the like. For example, it is only necessary to make the polysiloxane-covered ion-sustained-release glass flow in a dry state, disperse the acidic polymer solution from the upper part in the flowing state, and perform sufficiently stirring. Here, the method for dispersing the acidic polymer solution is not particularly limited, and a dropping or spraying method which enables uniform dispersion is more preferable.

The solvent that is used for preparing the acidic polymer solution used in the reaction is not particularly limited as long as it is a solvent in which the acidic polymer is soluble, and examples thereof comprise water, ethanol, acetone and the like. Among them, water is particularly preferable as water enables the acidic groups of the acidic polymer to be dissociated, resulting in uniform reaction with the polysiloxane-covered ion-sustained-release glass.

The weight average molecular weight of the acidic polymer dissolved in the acidic polymer solution may be, for example, 2000 to 50000, and is preferably 5000 to 40000. When the surface treatment is performed with an acidic polymer having a weight average molecular weight of 2000 or more, an acidic polymer reaction phase tends to be easily formed in the polysiloxane-covered ion-sustained-release glass, resulting in enhancement of ion-sustained-releasability. On the other hand, when the surface treatment is performed with an acidic polymer having a weight average molecular weight of 50000 or less, the viscosity of the acidic polymer solution does not become excessively high, and thus it is easy to uniformly treat the polysiloxane-covered ion-sustained-release glass. The acid polymer concentration in the acid polymer solution is, for example, 3 to 25 parts by weight, preferably 8 to 20 parts by weight. When the acidic polymer concentration is 3 parts by weight or more, the acidic polymer reaction phase described above is unlikely to become fragile, so that an effect of improving sustained release of ions is easily obtained. When the acidic polymer concentration is 25 parts by weight or less, the polysiloxane layer (porous) is easily diffused in a uniform state, a homogeneous acidic polymer reaction phase is easily obtained, and a reaction is unlikely to occur immediately after the contact with the polysiloxane coated ion-sustained-release glass, so that a problem such as generation of an intensely reacted aggregate hardly occurs. The amount of addition of the acidic polymer solution based on the amount of the polysiloxane-covered-ion-sustained-release glass may be 6 to 40 parts by weight, and is preferably 10 to 30 parts by weight. For example, when calculated from such amount of addition, the amount of the acidic polymer is preferably 1 to 7 parts by weight, and the amount of water is preferably 10 to 25 parts by weight, based on the amount of the polysiloxane-covered ion-sustained-release glass.

As the acidic polymer that can be used for forming an acidic polymer reaction phase on the surface of the polysiloxane-covered ion-sustained-release glass by the above-described method, any acidic polymer can be used as long as it is a copolymer or homopolymer derived from a polymerizable monomer having an acidic group (more specifically, at least one acidic group selected from the group consisting of a phosphoric acid residue, a pyrophosphoric acid residue, a thiophosphoric acid residue, a carboxylic acid residue, a sulfonic acid group and the like) can be used without any problem. Examples of the polymerizable monomer comprise acrylic acid, methacrylic acid, 2-chloroacrylic acid, 3-chloroacrylic acid, aconitic acid, mesaconic acid, maleic acid, itaconic acid, fumaric acid, glutaconic acid, citraconic acid, 4-(meth)acryloyloxyethoxycarbonylphthalic acid, 4-(meth)acryloyloxyethoxycarbonylphthalic anhydride, 5-(meth)acryloylaminopentylcarboxylic acid, 11-(meth)acryloyloxy-1,1-undecanedicarboxylic acid, 2-(meth)acryloyloxyethyl dihydrogen phosphate, 10-(meth)acryloyloxydecyl dihydrogen phosphate, 20-(meth)acryloyloxyecosyl dihydrogen phosphate, 1,3-di(meth)acryloyloxypropyl-2-dihydrogen phosphate, 2-(meth)acryloyloxyethylphenylphosphoric acid, 2-(meth)acryloyloxyethyl 2'-bromoethylphosphoric acid, (meth)acryloyloxyethyl phenyl phosphonate, di(2-(meth)acryloyloxyethyl) pyrophosphate, 2-(meth)acryloyloxyethyl dihydrogen dithiophosphate, 10-(meth)acryloyloxydecyl dihydrogen thiophosphate and the like. Among polymers obtained by (co)polymerizing such polymerizable monomers, homopolymers or copolymers derived from an $\alpha$-$\beta$ unsaturated carboxylic acid are preferable, in which an acid-base reaction with an acid-reactive element comprised in the polysiloxane-covered ion-sustained-release glass is relatively slow. Examples of such an $\alpha$-$\beta$ unsaturated carboxylic acid comprise acrylic acid polymers, acrylic acid-maleic acid copolymers, acrylic acid-itaconic acid copolymers and the like.

The ion-sustained-release glass is characterized by continual sustained release of ion species based on the glass composition, and whether or not the ion-sustained-release glass used for the spray powder mixture according to the present embodiment has ion-sustained-releasability can be determined by the following method.

Ion-sustained-release glass can be considered to have ion-sustained-releasability when F1 and F2 satisfies the relationship of the following expression (1) where F 1 is a concentration of ions released in a sustained manner in distilled water when 0.1 g of the ion-sustained-release glass is added to 100 g of the distilled water and the mixture is stirred for 1 hour, or a concentration of an element based on the ion species, and F2 is a concentration of ions released in a sustained manner in the distilled water when the mixture is stirred for 2 hours, or a concentration of the element based on the ion species.

$$F2 > F1 \quad \ldots \text{ expression (1)}$$

In the case where a plurality of ions released in a sustained manner from the ion-sustained-release glass, it is not necessary that the concentrations of all ions or elements based on the ion species satisfy the expression (1), and the ion-sustained-release glass can be considered to have ion-sustained-releasability when the concentration of at least one ion or element based on the ion species satisfies the expression (1).

[Optional Components other than Component (c)]

The spray powder mixture according to the present embodiment comprises the components (a) and (b). The spray powder mixture comprises optional components other than the component (c). Examples of the optional component comprise antibacterial agents that control the activity of bacteria, coloring materials that provides identifiability, and a fragrance that provides flavor and coolness. The spray powder mixture may contain these optional components as long as the effects of the present invention are not impaired.

Examples of the coloring material comprise inorganic white pigments (more specifically, titanium dioxide, zinc oxide and the like); inorganic red pigments (more specifically, iron oxide (colothar), iron titanate and the like); inorganic brown pigments (more specifically, γ-iron oxide and the like); inorganic yellow pigments (more specifically, yellow iron oxide, yellow soil and the like); inorganic black pigments (more specifically, black iron oxide, lower titanium oxide and the like); inorganic purple pigments (more specifically, mango violet, cobalt violet and the like); inorganic green pigments (more specifically, chromium oxide, chromium hydroxide, cobalt titanate and the like); inorganic blue pigments (more specifically, ultramarine blue, deep blue and the like); pearl pigments (more specifically, titanium oxide coated mica, titanium oxide coated bismuth oxychloride, titanium oxide coated talc, colored titanium oxide coated mica, bismuth oxychloride, fish scale foil and the like); metal powder pigments (more specifically, aluminum powder, copper powder and the like); organic pigments such as zirconium, barium and aluminum lake (more specifically, organic pigments such as Red No. 201, Red No. 202, Red No. 204, Red No. 205, Red No. 220, Red No. 226, Red No. 228, Red No. 405, Orange No. 203, Orange No. 204, Yellow No. 205, Yellow No. 401 and Blue No. 404; Red No. 3, Red No. 104, Red No. 106, Red No. 227, Red No. 230, Red No. 401, Red No. 505, Orange No. 205, Yellow No. 4, Yellow No. 5, Yellow No. 202, Yellow No. 203, Green No. 3, Blue No. 1 and the like); and natural dyes (more specifically, chlorophyll and β-carotene). These coloring materials can be used alone, or in combination of two or more thereof.

Examples of the antibacterial agent comprise benzoic acid, sodium benzoate, isopropyl parahydroxybenzoate, isobutyl parahydroxybenzoate, ethyl parahydroxybenzoate, methyl parahydroxybenzoate, butyl parahydroxybenzoate, propyl parahydroxybenzoate, isopropylmethylphenol, sodium sulfite, sodium hyposulfite, potassium pyrosulfite, sorbic acid, potassium sorbate, sodium dehydroacetate, piroctone olamine, thujaprisin, Aralia cordata extract, Styrax japonica extract, Artemisia capillaris extract, soft roe protein extract, enzymatically-decomposed pearl barley extract, bamboo extract, allylmustard oil, protamine, tea extract, grapefruit seed extract, lysozyme and chitosan and the like. These antibacterial agents can be used alone, or in combination of two or more thereof.

Examples of the fragrance comprise musk, lemon oil, 1-heptanol, α-methylionone, aldehyde C-10, aldehyde C-11, aldehyde C-9, allyl heptanoate, anisaldehyde, benzaldehyde, benzacetate, benzyl acetate, butyl propionate, cedar leaf oil, cedrol, cedryl acetate, cinnamic alcohol, cinnamon leaf, citronellal oil, citronellal, glove bad oil, cyclamen aldehyde, ethyl butyrate, ethyl caproate, ethyl isobutyrate, ethyl isovalerate, ethyl propionate, eucalyptus oil, eugenol, farnesol, geraniol, heptyl aldehyde, heptyl formate, hexyl acetate, hydrotropic aldehyde, isobonyl acetate, isoamyl formate, limonene, linalool, linalyl acetate, methyl heptenone, nonylaldehyde, organum oil, p-cresyl acetate, p-methylacetophenone, phenyl acetaldehyde, propyl propionate, spearmint oil, terpenyl acetate, linalool, tetrahydrolinalool, thymol, isobornyl acetate, α-ionone, β-ionone, acetyl cedrene, acetyl eugenol, alcohol C-10, alcohol C-11 undecylenic, alcohol C-12, aldehyde C-14, aldehyde C-18, anise alcohol, anisyl acetate, benzyl benzoate, benzyl isovalerate, benzyl salicylate, cinnamyl acetate, citronellol, citronellyl isobutyrate, citronellyl oxyacetaldehyde, coumarin, ethyl cinnamate, ethyl vanillin, geranyl isobutyrate, geranyltiglate, heliotropin, hexyl cinnamic aldehyde, hydroxycitronellal, indole, isoamyl cinnamic aldehyde, isoamyl salicylate, jasmone, methyl anthranilate, methyl cinnamate, muscone, musk ketone, nerolidol, pentalide, phenylacetic acid, lavender oil, menthol and vanillin and the like. These fragrances can be used alone, or in combination of two or more thereof.

(Method for producing Spray Powder Mixture)

The spray powder mixture according to the present embodiment can be produced by mixing the component (a), the component (b), and optional components if necessary. The method for mixing at least one selected from the group consisting of a saccharide, a sugar alcohol, an amino acid, a phosphoric acid compound, a carbonic acid compound and a calcium compound as the component (a) and hydrophobized particulate silica as the component (b) is not limited, and any mixing method can be used without any problem. The type of mixer used, the mixing method and conditions can be arbitrarily selected without limitation. In a preferred aspect, the primary particle diameter of the component (b) is much smaller than the size of the component (a). In such a case, the mixed state of these two components is not

particularly limited, any mixed state such as a coated state or a state of being dispersed in an aggregated form is acceptable, and mere existence of the two components in combination can produce the effects of the present invention.

For example, the effects described in the present specification are illustrative only, the present invention is not necessarily limited to these effects, and there may be additional effects.

For example, the spray powder mixture described in connection with the present embodiment comprises the component (a) and the component (b), and the presence of components that may be unavoidably or accidentally mixed during preparation, storage and/or use of the spray powder mixture (for example, components in an amount that can be recognized as being very small or extremely small, such as a very small amount or an extremely small amount of components) may be allowable.

The present invention is not limited to the above-described embodiments, and can be changed in design without departing from the spirit of the present invention.

## EXAMPLES

Hereinafter, the present invention will be specified and described in detail by way of examples, but the present invention is not limited to these examples. The unit of numerical values in Tables 2 to 4 is g (parts by mass).

<Provision of Spray Powder Mixture>

Materials used in examples and comparative examples and abbreviations thereof are shown below.

Component (a)

- Saccharide: trehalose and palatinose (disaccharide)
- Sugar alcohol: erythritol, reduced palatinose and mannitol (sugar alcohol)
- Carbonate compound: sodium hydrogen carbonate (sodium bicarbonate)

Component (b): particulate silica

- Particulate silica A: dimethylsilylated hydrophobic silica (primary particle diameter: 16 nm)
- Particulate silica B: trimethylsilylated hydrophobic silica (primary particle diameter: 12 nm)
- Particulate silica C: trimethylsilylated hydrophobic silica (primary particle diameter: 7 nm)
- Particulate silica D: non-hydrophobic silica (primary particle diameter: 12 nm)

Component (c): ion-sustained-release glass

- ion-sustained-release glass 1
- ion-sustained-release glass 2
- ion-sustained-release glass 3
- ion-sustained-release glass 4
- ion-sustained-release glass 5

Ion-sustained-release glasses 1 to 5 were prepared in accordance with the following procedure.

(Production of Ion-Sustained-Release Glass 1)

Various raw materials that are silicon dioxide, aluminum oxide, boron oxide, sodium fluoride and strontium carbonate were mixed to obtain a mixture. Thereafter, the obtained mixture was melted at 1400°C to obtain glass A (glass composition: $SiO_2$ at 22.5 mass%, $Al_2O_3$ at 20.0 mass%, $B_2O_3$ at 12.3 mass%, SrO at 35.7 mass%, $Na_2O$ at 2.5 mass% and F at 7.0 mass%). Next, the obtained glass A was ground for 100 hours using a vibrating mill. The obtained ground product was taken as ion-sustained-release glass A. 500 g of the ion-sustained-release glass A and 1105 g of a low-condensation silane compound ("MS51SG1" ($SiO_2$ content: 16%, polymerization degree: 2 to 6) manufactured by Mitsubishi Chemical Corporation) were put in a universal mixing stirrer, and mixed with stirring for 90 minutes. Thereafter, heat treatment was performed at 140°C for 30 hours to obtain a heat-treated product. The heat-treated product was crushed using a Henschel mixer to obtain polysiloxane-covered ion-sustained-release glass A. 500 g of the polysiloxane-covered glass A was collected and put in a Henschel mixer, and from the upper part thereof, an acidic polymer aqueous solution (polyacrylic acid aqueous solution having a polymer concentration of 13 parts by weight and a weight average molecular weight of 20000; manufactured by nacalai tesque) was sprayed while stirring was performed.

Thereafter, heat treatment was performed at 100°C for 3 hours to obtain ion-sustained-release glass 1. The obtained ion-sustained-release glass 1 was composite-surface-treated ion-sustained-release glass.

The mean particle diameter (D50) of the ion-sustained-release glass 1 was measured by a laser diffraction particle size measuring machine (Microtrac SPA: manufactured by Nikkiso Co., Ltd.), and the result showed that the mean particle diameter was 0.5 μm. The concentrations of elements based on various ions released from the composite-surface-treated ion-sustained-release glass 1 (the concentration of fluoride ions was converted tothe concentration of the fluorine element) were measured, and presence or absence of ion-sustained-release performance was determined from conformity to the expression (1). Table 1 shows the results.

(Production of Ion-Sustained-Release Glass 2)

Various raw materials that are silicon dioxide, aluminum oxide, boron oxide, sodium fluoride and strontium carbonate were mixed to obtain a mixture. Thereafter, the obtained mixture was melted at 1400°C to obtain glass B (glass composition: $SiO_2$ at 23.8 mass%, $Al_2O_3$ at 16.2 mass%, $B_2O_3$ at 10.5 mass%, SrO at 35.6 mass%, $Na_2O$ at 2.3 mass% and F at 11.6 mass%). Next, the obtained glass B was ground for 40 hours using a vibrating mill. The obtained ground product was taken as ion-sustained-release glass 2.

The mean particle diameter (D50) of the ion-sustained-release glass 2 was measured by a laser diffraction particle size measuring machine (Microtrac SPA: manufactured by Nikkiso Co., Ltd.), and the result showed that the mean particle diameter was 1.2 μm. The concentrations of elements based on various ions released in a sustained manner from the ion-sustained-release glass 2 (the concentration of fluoride ions was converted to the concentration of the fluorine element) were measured, and presence or absence of ion-sustained-release performance was determined from conformity with the expression (1). Table 1 shows the results.

(Production of Ion-Sustained-Release Glass 3)

500 g of the ion-sustained-release glass 2 and 1105 g of a low-condensation silane compound ("MS51SG1" ($SiO_2$ content: 16%, polymerization degree: 2 to 6) manufactured by Mitsubishi Chemical Corporation) were put in a universal mixing stirrer, and mixed with stirring for 90 minutes. Thereafter, heat treatment was performed at 140°C for 30 hours to obtain a heat-treated product. The heat-treated product was crushed using a Henschel mixer to obtain polysiloxane-covered ion-sustained-release glass 3. 500 g of the polysiloxane-covered ion-sustained-release glass 3 was collected and put in a Henschel mixer, and from the upper part thereof, an acidic polymer aqueous solution (polyacrylic acid aqueous solution having a polymer concentration of 13 parts by weight and a weight average molecular weight of 20000; manufactured by nacalai tesque) was sprayed while stirring was performed. Thereafter, heat treatment was performed at 100°C for 3 hours to obtain ion-sustained-release glass 3. The obtained ion-sustained-release glass 3 was composite-surface-treated ion-sustained-release glass.

The mean particle diameter (D50) of the ion-sustained-release glass 3 was measured by a laser diffraction particle size measuring machine (Microtrac SPA: manufactured by Nikkiso Co., Ltd.), and the result showed that the mean particle diameter was 1.3 μm. The concentrations of elements based on various ions released in a sustained manner from the composite-surface-treated ion-sustained-release glass 3 (the concentration of fluoride ions was converted to the concentration of the fluorine element) were measured, and presence or absence of ion-sustained-release performance was determined from conformity with the expression (1). Table 1 shows the results.

(Production of Ion-Sustained-Release Glass 4)

Various raw materials that are silicon dioxide, aluminum oxide, boron oxide, sodium fluoride and strontium carbonate were mixed to obtain a mixture. Thereafter, the obtained mixture was melted at 1400°C to obtain glass C (glass composition: $SiO_2$ at 19.8 mass%, $Al_2O_3$ at 19.8 mass%, $B_2O_3$ at 11.7 mass%, SrO at 35.0 mass%, $Na_2O$ at 2.3 mass% and F at 11.4 mass%). Next, the obtained glass C was ground for 10 hours using a vibrating mill. The obtained ground product was taken as ion-sustained-release glass C. 500 g of the ion-sustained-release glass C and 1660 g of a low-condensation silane compound ("MS51SG1" ($SiO_2$ content: 16%, polymerization degree: 2 to 6) manufactured by Mitsubishi Chemical Corporation) were put in a universal mixing stirrer, and mixed with stirring for 90 minutes. Thereafter, heat treatment was performed at 140°C for 30 hours to obtain a heat-treated product. The heat-treated product was crushed using a Henschel mixer to obtain polysiloxane-covered ion-sustained-release glass C. 500 g of the polysiloxane-covered glass B was collected and put in a Henschel mixer, and from the upper part thereof, an acidic polymer aqueous solution (polyacrylic acid aqueous solution having a polymer concentration of 13 parts by weight and a weight average molecular weight of 20000; manufactured by nacalai tesque) was sprayed while stirring was performed. Thereafter, heat treatment was performed at 100°C for 3 hours to obtain ion-sustained-release glass 4. The obtained ion-sustained-release glass 4 was composite-surface-treated ion-sustained-release glass.

The mean particle diameter (D50) of the ion-sustained-release glass 4 was measured by a laser diffraction particle size measuring machine (Microtrac SPA: manufactured by Nikkiso Co., Ltd.), and the result showed that the mean particle diameter was 3.1 μm. The concentrations of elements based on various ions released from the composite-surface-treated ion-sustained-release glass 4 (the concentration of fluoride ions was converted to the concentration of the fluorine element) were measured, and presence or absence of ion-sustained-release performance was determined from conformity with the expression (1). Table 1 shows the results.

(Production of Ion-Sustained-Release Glass 5)

Ion-sustained-release glass 5 was obtained by the same production method as that for the ion-sustained-release glass 4 except that the glass C was ground for 6 hours using a vibrating mill.
The mean particle diameter (D50) of the ion-sustained-release glass 5 was measured by a laser diffraction particle size measuring machine (Microtrac SPA: manufactured by Nikkiso Co., Ltd.), and the result showed that the mean particle diameter was 5.1 μm. The concentrations of elements based on various ions released from the composite-surface-treated ion-sustained-release glass 5 (the concentration of fluoride ions was converted to the concentration of the fluorine element) were measured, and the presence or absence of ion-sustained-release performance was determined from conformity with the expression (1). Table 1 shows the results.

Table 1

| | | | Ion-sustained-release glass | | | | |
|---|---|---|---|---|---|---|---|
| | | | Ion-sustained-release glass 1 | Ion-sustained-release glass 2 | Ion-sustained-release glass 3 | Ion-sustained-release glass 4 | Ion-sustained-release glass 5 |
| Surface treatment | | | Done | Not done | Done | Done | Done |
| Mean particle diameter (μm) | | | 0.5 | 1.2 | 1.3 | 3.1 | 5.1 |
| Element concentration (ppm) | F1 | F | 21.5 | 8.9 | 21.3 | 15.5 | 14.3 |
| | | B | 3.9 | 2.3 | 2.8 | 1.9 | 1.6 |
| | | Al | 0.3 | 0.2 | 0.1 | 0.1 | 0.1 |
| | | Sr | 15.5 | 9.2 | 14.6 | 9.5 | 8.9 |
| | F2 | F | 32.9 | 12.5 | 32.3 | 22.5 | 20.4 |
| | | B | 4.8 | 2.5 | 3.7 | 3.6 | 3.3 |
| | | Al | 0.4 | 0.3 | 0.2 | 0.05 | 0.03 |
| | | Sr | 21.8 | 9.7 | 21.3 | 12.4 | 10.9 |
| Conformity to F2 > F1 (expression 1) | | | ○ | ○ | ○ | ○ | ○ |
| Conform: O, not conform: × | | | | | | | |

<Preparation of Spray Powder Mixture>

(Examples 1 to 32 and Comparative Examples 1 to 6)

According to the compositions shown in Tables 2 to 4, the components were mixed to prepare spray powder mixtures of examples and comparative examples. As reference examples, commercially available powdered spray powder mixtures (AIRFLOW PLUS POWDER: EMS DENTAL, Perio-Mate Powder: NAKANISHI INC., Lunos: Dürr Dental SE) were used.

<Measurement Method and Evaluation Method>

Test methods (measurement method and evaluation method) employed in examples and comparative examples are as follows. Tables 1 and 5 to 7 show the results from the test methods.

[Evaluation of Ion-Sustained-Releasability of Ion-Sustained-Release Glass]

0.1 g of ion-sustained-release glass was added to 100 mL of distilled water, the mixture was stirred for 1 hour, and then filtered with a syringe filter for analysis (Chromatodisc 25 A, pore size: 0.2 μm; manufactured by GL Sciences Inc.), the concentration of an element based on each ion released in a sustained manner in the filtrate was measured, and the obtained measured value was taken as F1. 0.1 g of ion-sustained-release glass was added to 100 mL of distilled water, the mixture was stirred for 2 hours, and then similarly filtered with a syringe filter for analysis, the concentration of an element based on each ion released in a sustained manner in the filtrate was measured, and the obtained measured value was taken as F2. From the values of F1 and F2 (concentrations of the elements), conformity to the expression (1) was confirmed to determine the presence or absence of ion-sustained-releasability.

$$F2 > F1 \quad \ldots \text{ expression (1)}$$

Here, the specific measurement method of the element concentration is as follows. In the measurement of the concentration of the fluorine element, fluoride ions were measured using a fluorine ion composite electrode (Model 9609: manufactured by Orion Research Co., Ltd.) and an ion meter (Model 720 A: manufactured by Orion Research Co., Ltd.), and the measured value was used to convert to the concentration of the fluorine element. During measurement, 0.5 mL of TISABIII (manufactured by Orion Research Co., Ltd.) was added as an ionic strength adjusting agent. The calibration curve was prepared using 0.1, 1, 10 and 50 ppm standard solutions.

On the other hand, for measurement of other elements (B, Al and Sr), the concentrations of the elements were calculated by measurement using an inductively coupled plasma emission spectrometric analyzer (ICPS -8000: manufactured by Shimadzu Corporation). The calibration curve was prepared using 0, 10, 25 and 50 ppm standard solutions. In the case where the concentration of a measurement element was outside the calibration curve range, dilution was appropriately performed, followed by measurement.

[Evaluation of Fluidity of Spray Powder Mixture]

(Measurement of Repose Angle of Spray Powder Mixture)

The repose angle of the spray powder mixture prepared in each of examples and comparative examples was measured using a repose angle measuring device shown in Fig. 1, and was evaluated as an index of fluidity. Details are as described in the following test method.

-Test Method-

The spray powder mixture obtained in each of examples and comparative examples was put in a funnel portion of Fig. 1, and dropped in a sufficient amount so that the spray powder mixture spilled out from a stage. As a result, a mountain of the spray powder mixture deposited on the stage was formed. The inclination angle of the obtained mountain (Fig. 2) was measured using a special protractor. This operation was repeated twice, and an average of the obtained angles was calculated as a repose angle (°). The smaller the value of the repose angle, the better the fluidity of the spray powder mixture.

(Evaluation of Kinematic Fluidity of Spray Powder Mixture)

In accordance with the following test method, the repose angle of the spray powder mixture prepared in each of examples and comparative examples was evaluated as an index of fluidity.

-Test Method-

A certain amount (volume: 80 mL) of the spray powder mixture obtained in each of examples and comparative examples was put in a 100 mL plastic container together with a round stone (20 φ). The container was shaken up and down ten times, and allowed to stand upright on a desk. Immediately thereafter, the movement of the spray powder mixture in the plastic container was visually observed while the plastic container was slowly tilted by 90° was visually observed. From the obtained observation results, the kinematic fluidity of the spray powder mixture was evaluated in accordance with the following evaluation criteria.

-Evaluation Criteria-

⊙ (Very Good): After the plastic container inverts, the spray powder mixture moves until it is parallel to the desk top surface (more than 80 vol% of the spray powder mixture moves).
O (Good): After the plastic container inverts, the spray powder mixture moves (the spray powder mixture is not parallel to the desk top surface, and has an inclined surface, and 20 to 80 vol% of the spray powder mixture moves).
× (Poor): Even after the plastic container inverts, the spray powder mixture hardly moves (more than 80 vol% of the spray powder mixture does not move).

(Evaluation of Hydrophobicity of Powder Mixture)

In accordance with the following test method, the spray powder mixture prepared in each of examples and comparative examples was evaluated as an index of hydrophobicity.

-Test Method-

A spoonful (volume: 0.2 mL) of the spray powder mixture obtained in each of examples and comparative examples was taken, and put in water in a 50 mL glass container, and the mixture was stirred for 5 seconds using a plastic spatula. After the stirring, the state (appearance) of the introduced spray powder mixture was visually observed. From the obtained observation results, the hydrophobicity of the spray powder mixture was evaluated in accordance with the following evaluation criteria.

-Evaluation Criteria-

⊙ (Very Good): The spray powder mixture is not compatible with water at all, and 60 vol% or more of the spray powder mixture floats on the liquid surface.
O (Good): A part of the spray powder mixture is compatible with water, and more than 0 vol% and less than 60 vol% of the spray powder mixture floats on the liquid surface.
× (Poor): The spray powder mixture is compatible with water, all of the spray powder mixture is dispersed or dissolved in water, and there can be observed no floated spray mixture.

(Total Evaluation of Fluidity)

From the evaluation results for the repose angle, kinematic fluidity and hydrophobicity related to fluidity, total evaluation of the fluidity of the spray powder mixture was performed in accordance with the following evaluation criteria.

-Evaluation Criteria-

⊙ (Very Good): The repose angle is 40° or less, and both the evaluation results for kinematic fluidity and hydrophobicity are ⊙.
O (Good): The repose angle is 50° or less, at least one of the evaluation results for kinematic fluidity and hydrophobicity is O, and either of the evaluation results is not x.
x (Poor): The repose angle is more than 50°, and/or at least one of the evaluation results for kinematic fluidity and hydrophobicity is x.

[Evaluation of Low-Abrasive Property of Spray Powder Mixture]

(Measurement of Mean Particle Diameter of Spray Powder Mixture)

The mean particle diameter (μm) of the spray powder mixture prepared in each of examples and comparative examples by dry dispersion was measured using a laser diffraction particle size distribution measuring apparatus (Mastersizer 3000: manufactured by Malvern Panalytical).

In the spray powder mixtures of examples and comparative examples, the mean particle diameter of the spray powder mixture was substantially represented by the mean particle diameter of the component (a) because the content of the component (a) is (about 20 times or more) larger than that of the component (b), and the particle diameter of the component (a) is (about 1000 times or more) larger than that of the component (b).

(Evaluation of Degree of Aggregation of Spray Powder Mixture)

Evaluation was performed to confirm the state of the spray powder mixture including its aggregation state. Specifically, the particle size distribution of the spray powder mixture prepared in each of examples and comparative examples by dry dispersion was measured using a laser diffraction particle size distribution measuring apparatus (Mastersizer 3000: manufactured by Malvern Panalytical). The shape of the obtained particle size distribution was observed, and the number of peaks was counted. When two peaks were present, the volume ratio of particles in the spray powder mixture, which belong to the second peak, was calculated from the particle size distribution. From the number of peaks and the volume ratio in the obtained particle size distribution, the degree of aggregation of the spray powder mixture was evaluated according to the following criteria.

-Evaluation Criteria-

⊙ (Very Good): The number of peaks is 1, and aggregation is not observed at all.
O (Good): A second peak indicative of aggregation is present at 100 μm or more, and accounts for less than 10% on a volume basis.
× (Poor): A second peak indicative of aggregation is present at 100 μm or more, and accounts for 10% or more on a volume basis.

(Evaluation of Degree of Damage to Dentin by Spray Powder Mixture using Powder Spraying Apparatus)

The degree of damage to dentin by the spray powder mixture prepared in each of examples and comparative examples was evaluated in accordance with the following test method.

-Test Method-

The spray powder mixture obtained in each of example and comparative example was sprayed to the dentin surface at a spray distance of 3 mm and an angle of 45° under the conditions of a powder output of 50% and a water amount of 100% using a powder spraying apparatus (product name: AIRFLOW Master: manufactured by EMS DENTAL). The dentin surface after the treatment was observed using a desktop scanning electron microscope ("G2pro" manufactured by Phenom-World). From the observation results, the degree of damage to dentin by the spray powder mixture was evaluated in accordance with the following evaluation criteria. This operation was repeated three times to perform overall evaluation.

-Evaluation Criteria-

⊙ (Very Good): The dentin surface is not changed before and after the spraying treatment, and damage such as scratches is observed at all on the dentin surface.
O (Good): Damage such as scratches without irreguralities and peeling of dentin is observed on a part of the dentin surface.
△ (Fair): A part of the dentin surface after the spraying treatment has a rough surface (irregularities or the like), and damage such as scratches with peeling of dentin is observed on the dentin surface.
× (Poor): Damage such as scratches with irreguralities and peeling is observed on the dentin surface after the spraying treatment.

(Total Evaluation of Low-Abrasive Property)

From the evaluation results for the degree of aggregation and the degree of damage to dentin, total evaluation of the low-abrasive property was performed in accordance with the following evaluation criteria.

-Evaluation Criteria-

O (Very Good): Both the evaluation results for the degree of aggregation and the degree of damage to dentin are ⊙.
O (Good): The evaluation result for the degree of aggregation is O, or the evaluation result for the degree of damage to dentin is ○ or △, and either of the evaluation results is not ×.
x (Poor): At least one of the evaluation results for the degree of aggregation and the degree of damage to dentin is x.

[Overall Evaluation of Fluidity and Low-Abrasive Property]

From the total evaluation result for each of fluidity and the low-abrasive property, overall evaluation of fluidity and the low-abrasive property was performed in accordance with the following evaluation criteria.

-Evaluation Criteria-

⊙ (Very Good): Both the total evaluation results for fluidity and the low-abrasive property are ⊙.
O (Good): At least one of the total evaluation results for fluidity and the low-abrasive property is O, and either of the evaluation results is not ×.
x (Poor): At least one of the total evaluation results for fluidity and the low-abrasive property is x.

[Evaluation of Ion-Sustained-Releasability from Spray Powder Mixture]

The ion-sustained-releasability from the spray powder mixture prepared in each of examples and comparative examples was evaluated in accordance with the following test method.

-Test Method-

A certain amount (2.5 g) of the spray powder mixture obtained in each of examples and comparative examples was added to distilled water (15 mL) in a plastic container, and the mixture was stirred for 5 seconds using a plastic spatula. Thereafter, the liquid below the powder floated on the liquid surface was collected as a test liquid while the floated powder was avoided. The collected test liquid was filtered with a syringe filter for analysis (Chromatodisc 25 A, pore size: 0.2 μm; manufactured by GL Sciences Inc.), and the concentration of an element based on each ion released in a sustained manner and contained in the filtrate was measured.

Here, the specific measurement method of the element concentration is as follows. In the measurement of the concentration of the fluorine element, fluoride ions were measured using a fluorine ion composite electrode (Model 9609: manufactured by Orion Research Co., Ltd.) and an ion meter (Model 720 A: manufactured by Orion Research Co., Ltd.), and the measured value was used to calculate the concentration of the fluorine element. During measurement, 0.5 mL of TISABIII (manufactured by Orion Research Co., Ltd.) was added as an ionic strength adjusting agent. The calibration curve was prepared using 0.1, 1, 10 and 50 ppm standard solutions.

On the other hand, the concentrations of other elements (B, Al and Sr) were calculated by measurement using an inductively coupled plasma emission spectrometric analyzer (ICPS -8000: manufactured by Shimadzu Corporation). The calibration curve was prepared using 0, 1, 5 and 10 ppm standard solutions. In the case where the concentration of a measurement element was outside the calibration curve range, dilution was appropriately performed, followed by measurement.

[Table 2]

| | | Example 1 | Example 2 | Example 3 | Example 4 | Example 5 | Example 6 | Example 7 | Example 8 | Example 9 | Example 10 | Example 11 | Example 12 | Example 13 | Example 14 | Example 15 |
|---|---|---|---|---|---|---|---|---|---|---|---|---|---|---|---|---|
| Component (a) | Trehalose | 100 | | | | | | | | | | | | | | |
| | Palatinose | | | | | | | | | | | | | | | |
| | Erythritol | | 100 | | | | | | | | | | | | | |
| | Reduced palatinose | | | 100 | | | 100 | 100 | 100 | 100 | 100 | 100 | 100 | 100 | 100 | 100 |
| | Mannitol | | | | 100 | | | | | | | | | | | |
| | Sodium hydrogen carbonate | | | | | 100 | | | | | | | | | | |
| Component (b) | Particulate silica A | | | | | | 1 | 3 | 5 | | | | | | | |
| | Particulate silica B | | | | | | | | | 1 | 3 | 5 | | | | |
| | Particulate silica C | 1 | 1 | 1 | 1 | 1 | | | | | | | 0.1 | 0.5 | 3 | 5 |
| Particulate silica D that are not hydrophobized | | | | | | | | | | | | | | | | |
| Component (c) | Ion-sustained-release glass 1 | | | | | | | | | | | | | | | |
| | Ion-sustained-release glass 2 | | | | | | | | | | | | | | | |
| | Ion-sustained-release glass 3 | | | | | | | | | | | | | | | |
| | Ion-sustained-release glass 4 | | | | | | | | | | | | | | | |
| | Ion-sustained-release glass 5 | | | | | | | | | | | | | | | |

[Table 3]

| | | Example 16 | Example 17 | Example 18 | Example 19 | Example 20 | Example 21 | Example 22 | Example 23 | Example 24 | Example 25 | Example 26 | Example 27 | Example 28 | Example 29 |
|---|---|---|---|---|---|---|---|---|---|---|---|---|---|---|---|
| Component (a) | Trehalose | | | | | | | | | | | | | | |
| | Palatinose | | | | | | | | | | | | 100 | 100 | 100 |
| | Erythritol | | | | | | | | | | | | | | |
| | Reduced palatinose | 100 | 100 | 100 | 100 | 100 | 100 | | | | | | | | |
| | Mannitol | | | | | | | 100 | 100 | 100 | 100 | 100 | | | |
| | Sodium hydrogen carbonate | | | | | | | | | | | | | | |
| Component (b) | Particulate silica A | | | | | | | | | | | | | | |
| | Particulate silica B | 3 | 3 | | | | | 3 | 3 | 3 | 3 | 3 | | | |
| | Particulate silica C | | | 3 | 3 | 3 | 3 | | | | | | 3 | 3 | 3 |
| Particulate silica D that are not hydrophobized | | | | | | | | | | | | | | | |
| Component (c) | Ion-sustained-release glass 1 | | | | | | | 1 | | | | | | | |
| | Ion-sustained-release glass 2 | | | | | | | | 1 | | | | | | |
| | Ion-sustained-release glass 3 | 1 | 3 | 1 | 3 | 5 | 10 | | | 1 | | | | 1 | 3 |
| | Ion-sustained-release glass 4 | | | | | | | | | | 1 | | | | |
| | Ion-sustained-release glass 5 | | | | | | | | | | | 1 | | | |

[Table 4]

| | | Example 30 | Example 31 | Example 32 | Comparative Example 1 | Comparative Example 2 | Comparative Example 3 | Comparative Example 4 | Comparative Example 5 | Comparative Example 6 | Reference Example 1 | Reference Example 2 | Reference Example 3 |
|---|---|---|---|---|---|---|---|---|---|---|---|---|---|
| Component (a) | Trehalose | | | | | | | | | | AIRFLOW PLUS POWDER | Perio-Mate Powder | Lunos |
| | Palatinose | | | | | | | | | | | | |
| | Erythritol | | | | | | | | | | | | |
| | Reduced palatinose | 100 | 100 | 100 | 100 | 100 | 100 | 100 | 100 | 100 | | | |
| | Mannitol | | | | | | | | | | | | |
| | Sodium hydrogen carbonate | | | | | | | | | | | | |
| Component (b) | Particulate silica A | 1 | 3 | 5 | | | | | | | | | |
| | Particulate silica B | | | | | | | | | | | | |
| | Particulate silica C | | | | | | | | | | | | |
| Particulate silica D that are not hydrophobized | | | | | | 1 | 3 | 5 | 3 | 3 | | | |
| Component (c) | Ion-sustained-release glass 1 | 3 | 3 | 3 | | | | | | | | | |
| | Ion-sustained-release glass 2 | | | | | | | | | | | | |
| | Ion-sustained-release glass 3 | | | | | | | | 1 | 3 | | | |
| | Ion-sustained-release glass 4 | | | | | | | | | | | | |
| | Ion-sustained-release glass 5 | | | | | | | | | | | | |

For the prepared powder mixture composition and the commercially available spray powder mixtures, the repose angle, kinematic fluidity, hydrophobicity, the particle size, the degree of damage to dentine, and ion-sustained-releasability property were evaluated in accordance with the above-described methods. Tables 5 to 7 shows the results of these tests.

[Table 5]

| | Fluidity | | | | Low-abrasive property | | | | Overall evaluation | Ion-sustained-releasability (ppm) | | | |
|---|---|---|---|---|---|---|---|---|---|---|---|---|---|
| | | | | | Particle size | | | | | | | | |
| | Repose angle (°) | Kinematic fluidity | Hydrophobic | Total evaluation | Mean particle diameter (μm) | Degree of aggregation | Damage to dentin | Total evaluation | | B | Al | Sr | F |
| Example 1 | 33 | ○ | ⊙ | ○ | 30.2 | ○ | ○ | ○ | ○ | 0.0 | 0.0 | 0.0 | 0.0 |
| Example 2 | 31 | ⊙ | ⊙ | ⊙ | 23.6 | ⊙ | ⊙ | ⊙ | ⊙ | 0.0 | 0.0 | 0.0 | 0.0 |
| Example 3 | 32 | ⊙ | ⊙ | ⊙ | 27.0 | ⊙ | ⊙ | ⊙ | ⊙ | 0.0 | 0.0 | 0.0 | 0.0 |
| Example 4 | 35 | ⊙ | ⊙ | ⊙ | 20.1 | ⊙ | ⊙ | ⊙ | ⊙ | 0.0 | 0.0 | 0.0 | 0.0 |
| Example 5 | 29 | ⊙ | ⊙ | ⊙ | 65.5 | ⊙ | Δ | ○ | ○ | 0.0 | 0.0 | 0.0 | 0.0 |
| Example 6 | 44 | ○ | ○ | ○ | 25.0 | ○ | ⊙ | ○ | ○ | 0.0 | 0.0 | 0.0 | 0.0 |
| Example 7 | 43 | ○ | ○ | ○ | 30.6 | ○ | ⊙ | ○ | ○ | 0.0 | 0.0 | 0.0 | 0.0 |
| Example 8 | 41 | ○ | ⊙ | ⊙ | 33.4 | ○ | ⊙ | ○ | ○ | 0.0 | 0.0 | 0.0 | 0.0 |
| Example 9 | 31 | ⊙ | ⊙ | ⊙ | 26.5 | ⊙ | ⊙ | ⊙ | ⊙ | 0.0 | 0.0 | 0.0 | 0.0 |
| Example 10 | 34 | ⊙ | ⊙ | ⊙ | 26.8 | ⊙ | ⊙ | ⊙ | ⊙ | 0.0 | 0.0 | 0.0 | 0.0 |
| Example 11 | 34 | ⊙ | ⊙ | ⊙ | 27.2 | ⊙ | ⊙ | ⊙ | ⊙ | 0.0 | 0.0 | 0.0 | 0.0 |
| Example 12 | 50 | ○ | ○ | ○ | 28.3 | ○ | ○ | ○ | ○ | 0.0 | 0.0 | 0.0 | 0.0 |
| Example 13 | 33 | ⊙ | ⊙ | ⊙ | 27.4 | ⊙ | ⊙ | ⊙ | ⊙ | 0.0 | 0.0 | 0.0 | 0.0 |

(continued)

| | Fluidity | | | | Low-abrasive property | | | | Overall evaluation | Ion-sustained-releasability (ppm) | | | |
|---|---|---|---|---|---|---|---|---|---|---|---|---|---|
| | | | | | Particle size | | Damage to dentin | Total evaluation | | | | | |
| | Repose angle (°) | Kinematic fluidity | Hydrophobic | Total evaluation | Mean particle diameter (μm) | Degree of aggregation | | | | B | Al | Sr | F |
| Example 14 | 33 | ⊙ | ⊙ | ⊙ | 26.0 | ⊙ | ⊙ | ⊙ | ⊙ | 0.0 | 0.0 | 0.0 | 0.0 |
| Example 15 | 34 | ⊙ | ⊙ | ⊙ | 24.7 | ⊙ | ⊙ | ⊙ | ⊙ | 0.0 | 0.0 | 0.0 | 0.0 |

[Table 6]

| | Hydrophobic | | | | Low-abrasive property | | | | Overall evaluation | Ion-sustained-releasability (ppm) | | | |
|---|---|---|---|---|---|---|---|---|---|---|---|---|---|
| | | | | | Particle size | | | | | | | | |
| | Repose angle (°) | Kinematic fluidity | Hydrophobic | Total evaluation | Mean particle diameter (μm) | Degree of aggregation | Damage to dentin | Total evaluation | | B | Al | Sr | F |
| Example 16 | 33 | ⊙ | ⊙ | ⊙ | 25.0 | ⊙ | ⊙ | ⊙ | ⊙ | 2.0 | 0.4 | 4.8 | 1.5 |
| Example 17 | 40 | ⊙ | ⊙ | ⊙ | 26.7 | ⊙ | ⊙ | ⊙ | ⊙ | 4.7 | 1.4 | 10.1 | 3.7 |
| Example 18 | 30 | ⊙ | ⊙ | ⊙ | 25.5 | ⊙ | ⊙ | ⊙ | ⊙ | 2.0 | 0.4 | 4.4 | 1.5 |
| Example 19 | 39 | ⊙ | ⊙ | ⊙ | 25.9 | ⊙ | ⊙ | ⊙ | ⊙ | 5.0 | 1.6 | 11.2 | 4.0 |
| Example 20 | 40 | ⊙ | ⊙ | ⊙ | 27.4 | ⊙ | ⊙ | ⊙ | ⊙ | 8.7 | 2.2 | 17.9 | 7.4 |
| Example 21 | 44 | ○ | ⊙ | ○ | 27.8 | ○ | ○ | ○ | ○ | 16.1 | 5.5 | 27.8 | 13.9 |
| Example 22 | 35 | ⊙ | ⊙ | ⊙ | 20.3 | ⊙ | ⊙ | ⊙ | ⊙ | 2.5 | 0.5 | 5.3 | 1.8 |
| Example 23 | 33 | ⊙ | ⊙ | ⊙ | 21.1 | ⊙ | ⊙ | ⊙ | ⊙ | 1.3 | 0.3 | 2.1 | 0.9 |
| Example 24 | 33 | ⊙ | ⊙ | ⊙ | 22.9 | ⊙ | ⊙ | ⊙ | ⊙ | 1.9 | 0.4 | 4.6 | 1.6 |
| Example 25 | 32 | ⊙ | ⊙ | ⊙ | 23.0 | ⊙ | ⊙ | ⊙ | ⊙ | 1.6 | 0.3 | 4.0 | 1.4 |
| Example 26 | 30 | ⊙ | ⊙ | ⊙ | 24.5 | ⊙ | ○ | ○ | ○ | 1.4 | 0.2 | 3.8 | 1.2 |
| Example 27 | 38 | ○ | ⊙ | ○ | 31.4 | ⊙ | ○ | ○ | ○ | 0.0 | 0.0 | 0.0 | 0.0 |

(continued)

| | Hydrophobic | | | | Low-abrasive property | | | | Overall evaluation | Ion-sustained-releasability (ppm) | | | |
|---|---|---|---|---|---|---|---|---|---|---|---|---|---|
| | | | | | Particle size | | | | | | | | |
| | Repose angle (°) | Kinematic fluidity | Hydrophobic | Total evaluation | Mean particle diameter (μm) | Degree of aggregation | Damage to dentin | Total evaluation | | B | Al | Sr | F |
| Example 28 | 37 | ○ | ⊙ | ○ | 33.0 | ○ | ○ | ○ | ○ | 1.8 | 0.3 | 4.0 | 1.2 |
| Example 29 | 43 | ○ | ⊙ | ○ | 34.2 | ○ | ○ | ○ | ○ | 4.8 | 1.5 | 10.3 | 4.0 |

[Table 7]

| | Fluidity | | | | Low-abrasive property | | | | Overall evaluation | Ion-sustained-releasability (ppm) | | | |
|---|---|---|---|---|---|---|---|---|---|---|---|---|---|
| | | | | | Particle size | | | | | | | | |
| | Repose angle (°) | Kinematic fluidity | Hydrophobic | Total evaluation | Mean particle diameter (μm) | Degree of aggregation | Damage to dentin | Total evaluation | | B | Al | Sr | F |
| Example 30 | 48 | ○ | ○ | ○ | 26.8 | ○ | ⊙ | ○ | ○ | 5.5 | 1.8 | 12.4 | 4.5 |
| Example 31 | 46 | ○ | ○ | ○ | 33.3 | ○ | ⊙ | ○ | ○ | 4.8 | 1.3 | 11.4 | 43 |
| Example 32 | 46 | ○ | ○ | ○ | 34.1 | ○ | ⊙ | ○ | ○ | 4.4 | 1.5 | 11.9 | 43 |
| Comparative Example 1 | 57 | × | × | × | 28.9 | × | ○ | × | × | 0.0 | 0.0 | 0.0 | 0.0 |
| Comparative Example 2 | 47 | × | × | × | 25.0 | ○ | ○ | ○ | × | 0.0 | 0.0 | 0.0 | 0.0 |
| Comparative Example 3 | 43 | × | × | × | 23.9 | ○ | ○ | ○ | × | 0.0 | 0.0 | 0.1 | 0.0 |
| Comparative Example 4 | 45 | × | × | × | 22.4 | ○ | ○ | ○ | × | 0.0 | 0.0 | 0.0 | 0.0 |
| Comparative Example 5 | 44 | × | × | × | 24.7 | ○ | ○ | ○ | × | 3.8 | 1.3 | 11.9 | 4.6 |
| Comparative Example 6 | 47 | × | × | × | 26.3 | × | ○ | × | × | 8.4 | 2.5 | 20.5 | 7.6 |
| Reference Example 1 | 31 | ⊙ | ○ | ○ | 15.3 | ○ | | - - | | | - - | - - | - |
| Reference Example 2 | 27 | ⊙ | ○ | ○ | 30.1 | × | | - - | | | - - | - - | - |
| Reference Example 3 | 40 | ⊙ | ○ | ○ | 43.7 | × | - | - | | | - | - | - |

As shown in Tables 2 to 4, the spray powder mixtures of Examples 1 to 32 comprise both the component (a) and the component (b). Therefore, the spray powder mixtures of Examples 1 to 32 are included in the scope of the invention according to claim 1.

As shown in Tables 5 to 7, the spray powder mixtures of Examples 1 to 32 were rated ⊙ or ○ in the overall evaluation of fluidity and the low-abrasive property.

As shown in Table 4, any of Comparative Examples 1 to 6 does not comprise the component (b). Specifically, the spray powder mixture of Comparative Example 1 comprises only the component (a), and does not comprise the hydrophobized particulate silica as the component (b). The spray powder mixtures of Comparative Examples 2 to 4 comprise the component (a) and particulate silica that is not hydrophobized. The spray powder mixtures of Comparative Examples 5 and 6 comprise the component (a), particulate silica that is not hydrophobized, and ion-sustained release glass. Therefore, the spray powder mixtures of Comparative Examples 1 to 6 are outside the scope of the invention according to claim 1.

As shown in Table 7, the spray powder mixtures of Comparative Examples 1 to 7 were all rated × in the overall evaluation of fluidity and the low-abrasive property.

Therefore, it is evident that the spray powder mixtures of examples are superior in both fluidity and low-abrasive property to the spray powder mixtures of the comparative examples.

As shown in Tables 3 and 4, the spray powder mixtures of Examples 16 to 26 and 28 to 32 further comprise the component (c) in addition to the component (a) and the component (b). Therefore, the spray powder mixtures of Examples 16 to 26 and 28 to 32 are included in the invention according to claims 2 and 3.

For the spray powder mixtures of Examples 16 to 26 and 28 to 32, the concentrations of the elements of B, Al, Sr and F were measured as shown in Tables 6 and 7. As a result, sustained release of ions having B, Al, Sr and F as constituent elements was confirmed.

As shown in Tables 2 and 3, the spray powder mixtures of Examples 1 to 15 and 28 comprisethe component (a) and the component (b), but do not comprise the component (c). The spray powder mixtures of Examples 1 to 15 and 27 are included in the scope of the invention according to claim 1, but are outside the scope of the invention according to claims 2 and 3.

For the spray powder mixtures of Examples 1 to 15 and 27, the concentrations of the elements of B, Al, Sr and F were measured as shown in Tables 5 and 6. As a result, sustained release of ions having B, Al, Sr and F as constituent elements was not confirmed.

Therefore, it is evident that the spray powder mixtures of Examples 16 to 26 and 27 to 32 are further excellent in both fluidity and low-abrasive property, and have ion-sustained-releasability as compared to the spray powder mixtures of Examples 1 to 15 and 27. Thus, it may be possible to exhibit strengthening of the tooth, suppression of the activity of bacteria, and the like for the tooth above and below the gingival margin, the soft tissues in the gingival sulcus and the periodontal pocket, and the like by the effect of sustained release of ions.

[Cross-Reference to Related Application]

The present application claims priority based on Japanese Patent Application No. 2020-205830 filed on December 11, 2020, the entire contents of which are incorporated herein by reference.

## INDUSTRIAL APPLICABILITY

For example, the spray powder mixture of the present invention can be stably sprayed to clean the tooth surface above or below the gingival margin or the inside of gingival sulcus and the periodontal pocket by uniform mixing with air in a powder/air mixing chamber of a powder spraying apparatus, and can efficiently remove calculus and plaque firmly stuck on the tooth surface without damaging the tooth surface, and achieve strengthening of the tooth and suppression of the activity of bacteria for the tooth above and below the gingival margin, the hard and soft tissues in the gingival sulcus and the periodontal pocket, and the like by the effect of sustained release of ions.

## Claims

**1.** A spray powder mixture to be sprayed to a tooth surface above or below a gingival margin or into a gingival sulcus and a periodontal pocket by a powder spraying apparatus, the spray powder mixture comprising:

(a) at least one component selected from a group consisting of a saccharide, a sugar alcohol, an amino acid, a phosphate compound, a carbonate compound, and a calcium compound; and
(b) a hydrophobized particulate silica.

2. The spray powder mixture according to claim 1, further comprising (c) ion-sustained-release glass.

3. The spray powder mixture according to claim 2, wherein the (c) ion-sustained-release glass is capable of releasing at least any one of a fluorine ion, a strontium ion, a borate ion, and an aluminum ion in a sustained manner.

4. The spray powder mixture according to any one of claims 1 to 3, wherein the component of the (a) is a sugar alcohol.

5. The spray powder mixture according to any one of claims 1 to 4, wherein the component of the (a) is a sugar alcohol that is a reduced disaccharide.

6. The spray powder mixture according to claim 4 or 5, wherein a solubility of the sugar alcohol in water at 20°C is 30 wt% or less.

7. The spray powder mixture according to any one of claims 1 to 6, wherein the hydrophobized particulate silica of the (b) is a trialkylsilylated particulate silica.

FIG. 1

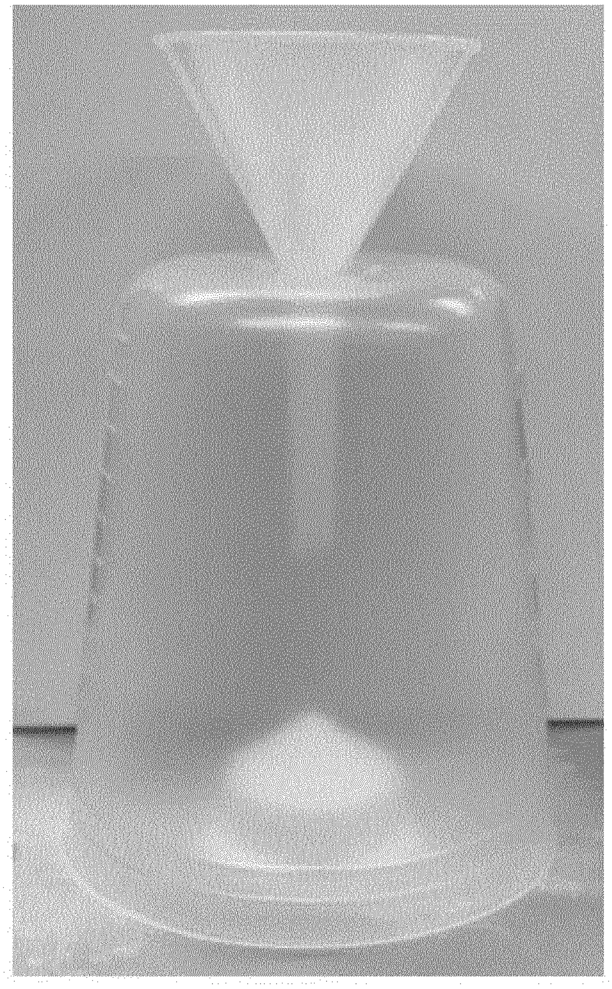

FIG. 2

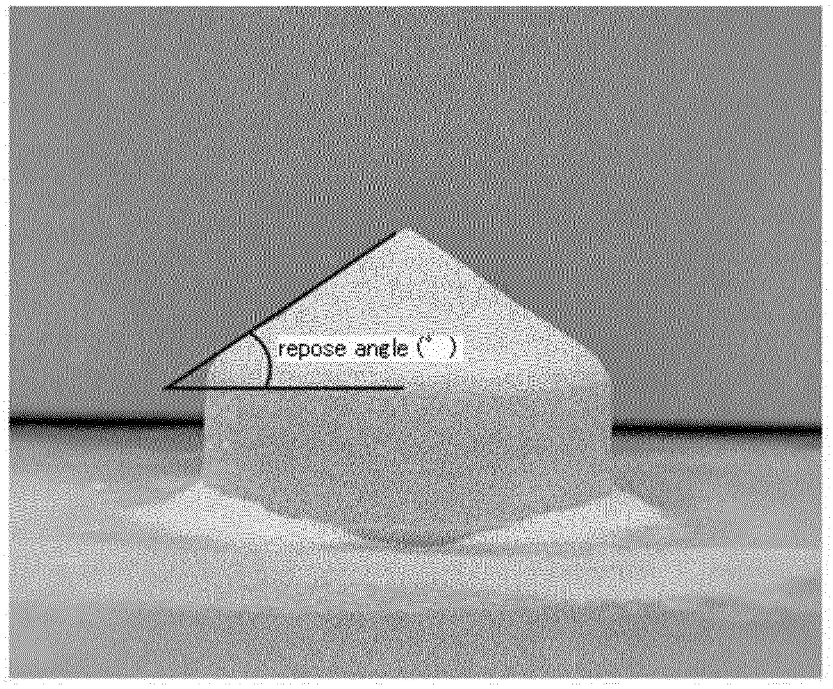

**INTERNATIONAL SEARCH REPORT**

International application No. **PCT/JP2021/042276**

**A. CLASSIFICATION OF SUBJECT MATTER**

***A61Q 11/00***(2006.01)i; ***A61K 8/02***(2006.01)i; ***A61K 8/19***(2006.01)i; ***A61K 8/25***(2006.01)i; ***A61K 8/34***(2006.01)i; ***A61K 8/44***(2006.01)i; ***A61K 8/60***(2006.01)i
FI: A61K8/60; A61K8/34; A61K8/44; A61K8/25; A61Q11/00; A61K8/19; A61K8/02

According to International Patent Classification (IPC) or to both national classification and IPC

**B. FIELDS SEARCHED**

Minimum documentation searched (classification system followed by classification symbols)

A61Q11/00; A61K8/00-8/99; A61C7/00; A61C3/025

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X<br>Y | JP 2003-528127 A (3M ESPE AG) 24 September 2003 (2003-09-24)<br>claims, paragraphs [0028], [0030], [0032], [0051]-[0058] | 1, 7<br>1-7 |
| Y | JP 2010-215621 A (FERTON HOLDING SA) 30 September 2010 (2010-09-30)<br>claims, paragraphs [0013]-[0041] | 1-7 |
| Y | JP 2015-227311 A (SHOFU INC.) 17 December 2015 (2015-12-17)<br>claims, paragraphs [0001], [0037], [0041], [0050]-[0058] | 2-7 |
| Y | JP 2009-539755 A (IMPERIAL INNOVATIONS LTD.) 19 November 2009 (2009-11-19)<br>paragraphs [0069]-[0073] | 2-7 |
| Y | JP 2011-526298 A (NOVAMIN TECHNOLOGY, INC.) 06 October 2011 (2011-10-06)<br>claims | 2-7 |
| A | JP 11-244303 A (MORITA MFG. CO., LTD.) 14 September 1999 (1999-09-14) | 1-7 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **13 January 2022** | **25 January 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2021/042276**

| Patent document cited in search report | Publication date (day/month/year) | Patent family member(s) | Publication date (day/month/year) |
|---|---|---|---|
| JP 2003-528127 A | 24 September 2003 | US 2004/0091429 A1<br>claims, paragraphs [0028], [0030], [0032], [0051]-[0057], table 1<br>WO 2001/072273 A2<br>EP 1265589 A2 | |
| JP 2010-215621 A | 30 September 2010 | US 2010/0297576 A1<br>claims, paragraphs [0016]-[0045]<br>EP 2228175 A1 | |
| JP 2015-227311 A | 17 December 2015 | (Family: none) | |
| JP 2009-539755 A | 19 November 2009 | US 2009/0208428 A1<br>paragraphs [0069]-[0073]<br>WO 2007/144662 A1<br>EP 2037973 A1<br>KR 10-2009-0037889 A<br>CN 101500622 A | |
| JP 2011-526298 A | 06 October 2011 | US 2009/0324516 A1<br>claims<br>WO 2009/158564 A1<br>EP 2306961 A1<br>CN 102083404 A | |
| JP 11-244303 A | 14 September 1999 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4174571 A
- US 4595365 A
- JP 4418113 A
- US 6126444 A
- US 5810587 A
- JP 5846720 A
- JP 4418113 B
- JP 5846720 B
- JP 2020205830 A